# EUROPEAN PATENT APPLICATION

(11) **EP 4 737 565 A1**
(43) Date of publication of application: **06.05.2026**
(21) Application number: 24210156.6
(22) Date of filing: 31.10.2024
(51) Int. Cl.: C12N 9/12, C12N 9/24, C12P 5/00, C12R 1/01

(54) **UTILIZATION OF SUCROSE FOR INCREASED PRODUCTION OF TERPENES IN RHODOBACTER MICROBIAL CELL FACTORIES**

(71) Applicant: Isobionics B.V., 6167 RD Geleen (NL); Stichting Wageningen Research, 6708 PB Wageningen (NL)
(72) Inventor: Cankar, Katarina, 6708 PB Wageningen (NL); Van Houwelingen, Adele Margaretha Maria Liduina, 6708 PB Wageningen (NL); Bosch, Hendrik Jan, 6708 PB Wageningen (NL); Beekwilder, Martinus Julius, 6167 RD Geleen (NL)
(74) Representative: Altmann Stößel Dick Patentanwälte PartG mbB

(57) **Abstract**

The present invention relates to a Rhodobacter cell engineered to utilize sucrose, comprising: (i) a heterologous enzyme having invertase activity; (ii) a heterologous enzyme having sucrose permease activity; and, optionally, (iii) a heterologous enzyme having fructokinase activity, and to a method for producing a Rhodobacter cell engineered to utilize sucrose. The invention further relates to a method for preparing a monoterpene, a sesquiterpene, a diterpene, a triterpene, a tetraterpene, a flavour, an aroma compound, an UV scavenger, a natural colorant, a natural crop protectant or an isoprenoid moiety containing compound such as coenzyme Q10, comprising culturing the Rhodobacter cell according to the invention in a culture medium comprising sucrose as carbon source for the monoterpene, sesquiterpene, diterpene, triterpene, tetraterpene, flavour, aroma compound, UV scavenger, natural colorant, natural crop protectant or the isoprenoid moiety containing compound such as coenzyme Q10. In addition, the invention pertains to the use of the Rhodobacter cell of the invention for the production of monoterpenes, sesquiterpenes, diterpenes, triterpenes, tetraterpenes, flavours, aroma compounds, UV scavengers, natural colorants, natural crop protectants or for production of isoprenoid moiety containing compounds such as coenzyme Q10.

## Description

The present invention relates to a Rhodobacter cell engineered to utilize sucrose, comprising: (i) a heterologous enzyme having invertase activity; (ii) a heterologous enzyme having sucrose permease activity; and, optionally, (iii) a heterologous enzyme having fructokinase activity, and to a method for producing a Rhodobacter cell engineered to utilize sucrose. The invention further relates to a method for preparing a monoterpene, a sesquiterpene, a diterpene, a triterpene, a tetraterpene, a flavour, an aroma compound, an UV scavenger, a natural colorant, a natural crop protectant or an isoprenoid moiety containing compound such as coenzyme Q10, comprising culturing the Rhodobacter cell according to the invention in a culture medium comprising sucrose as carbon source for the monoterpene, sesquiterpene, diterpene, triterpene, tetraterpene, flavour, aroma compound, UV scavenger, natural colorant, natural crop protectant or the isoprenoid moiety containing compound such as coenzyme Q10. In addition, the invention pertains to the use of the Rhodobacter cell of the invention for the production of monoterpenes, sesquiterpenes, diterpenes, triterpenes, tetraterpenes, flavours, aroma compounds, UV scavengers, natural colorants, natural crop protectants or for production of isoprenoid moiety containing compounds such as coenzyme Q10.

Carbon source is one of the major cost drivers for industrial production of bulk chemicals from microbial fermentation. Currently, glucose, typically from corn, is the most common carbon source for industrial fermentation in Escherichia coli. Sucrose from sugarcane or sugarbeet, however, would be preferable to corn-based glucose as a carbon substrate for E. coli-based industrial fermentation. First, it is a cheaper substrate as it can be directly fermented, either as cane juice or as molasses, or it can be easily made into pure sugar by high-temperature crystallization, whereas glucose has to be converted from starch by milling and enzymatic hydrolysis. Second, sugarcane sucrose-based bioprocesses are more environmentally friendly and sustainable than glucose-based bioprocesses. This is because bagasse, the fibrous by-product from sugarcane mills, can be utilized to produce energy for the bioprocess, whereas corn glucose-based processes rely on fossil fuels for energy. Finally, as a result of these two primary factors, the associated overall bioprocess cost is decreased relative to that with glucose. In addition, sucrose is highly abundant and readily available.

The ability to metabolize sucrose as a carbon source is a highly variable feature among E. coli strains. Sucrose-fermenting strains include the enteropathogenic strains, B-62, EC3132 and its mutants, and E. coli W (Archer C, Kim J, Jeong H, Park JH, Vickers CE, Lee SY, Nielsen LK. 2011. The genome sequence of E. coli W (ATCC 9637): comparative genome analysis and an improved genome-scale reconstruction of E. coli. BMC Genomics 12:9; V.B. Shukla, S. Zhou, L.P. Yomano, K.T. Shanmugam, J.F. Preston & L.O. Ingram. Production of D(-)-lactate from sucrose and molasses. Biotechnology Letters 26: 689-693, 2004). There are two gene clusters responsible for sucrose catabolism in E. coli: the scr regulon, encoding a sucrose phosphotransferase system (PTS), and the chromosomally carried sucrose catabolism (csc) regulon, encoding a sucrose non-PTS utilization system (Jahreis K, Bentler L, Bockmann J, Hans S, Meyer A, Siepelmeyer J, Lengeler JW. 2002. Adaptation of sucrose metabolism in the Escherichia coli wild-type strain EC3132. J. Bacteriol. 184:5307-5316). Additionally, the E. coli KO11, an ethanologenic derivative of the B strain, has the native ability to ferment sucrose (Moniruzzaman M, Lai X, York SW, Ingram LO (1997) Extracellular melibiose and fructose are intermediates in raffinose catabolism during fermentation to ethanol by engineered enteric bacteria J. Bacteriol. 179: 1880-1886). Genomic DNA from this organism was used to construct a gene library. Transformants of E. coli DH5α were selected for ampicillin resistance and screened for sucrose fermentation using selection plates containing 1% sucrose. Three stable clones growing on sucrose were identified and one was fully sequenced (GenBank accession number AY314757) revealing that the clone contained three complete open reading frames encoding invertase (cscA), and a bicistronic operon (cscKB) encoding fructokinase and an anion symporter for sucrose, respectively.

In one study, Bruschi et al. describe the transfer of the csc operon into non-sucrose utilizing E. coli strains (Bruschi M, Boyes SJ, Sugiarto H, Nielsen LK, Vickers CE. 2012. A transferable sucrose utilization approach for non-sucrose-utilizing Escherichia coli strains. Biotechnol. Adv. 30:1001-1010).

In the study of Shukla et al. (loc. cit.), the sucrose utilization genes (truncated cscR, cscA and cscKB) from the csc regulon from *E. coli* KO11 were transferred to non-sucrose utilizing E. coli W3110 derivatives, strains SZ63 and SZ85. The resulting plasmid was functionally expressed in SZ63 and enabled growth on sucrose, but was unstable in strain SZ85.

The roles of the chromosomally encoded sucrose catabolism (csc) genes in E. coli W were examined by knockout and overexpression experiments, in a study by Sabri et al. (Suriana Sabri, Lars K. Nielsen, and Claudia E. Vickers; Appl Environ Microbiol. 2013 Jan; 79(2): 478-487)). It was found that at low sucrose concentrations, the csc genes were repressed and cells could not grow. Removal of either the repressor protein (cscR) or the fructokinase (cscK) gene facilitated de-repression. Furthermore, combinatorial knockout of cscR and cscK conferred an improved growth rate on low sucrose. The invertase (cscA) and sucrose permease (cscB) genes were essential for sucrose catabolism in E. coli W, demonstrating that no other genes can provide sucrose transport or inversion activities. However, cscK was not essential for sucrose utilization.

Loewe et al. demonstrate engineering of the csc operon from E. coli into Pseudomonas putida and Cupriavidus necator to enable sucrose utilization, in particular the invertase (Löwe H, Schmauder L, Hobmeier K, Kremling A, Pflüger-Grau K. Microbiologyopen. 2017 Aug;6(4):e00473. doi: 10.1002/mbo3.473. Epub 2017 Mar 27).

Arikawa et al. describe introducing the csc operon in Cupriavidus necator for polyhydroxyalka-noate production from sucrose (Arikawa H, Matsumoto K, Fujiki T. Appl Microbiol Biotechnol. 2017 Oct; 101 (20):7497-7507. doi: 10.1007/s00253-017-8470-7. Epub 2017).

Rhodobacter is a robust and established industrial microorganism that is currently used to produce and commercialize natural terpene aromas for the flavour and fragrance market. The current process uses glucose as carbon source. A fermentation process using sucrose as carbon source instead of glucose could lead to a more cost-efficient terpene production. Though some microbes can efficiently utilize sucrose as a carbon source, Rhodobacter naturally metabolises sucrose very poorly. In one study, Sun et al. demonstrate growth of non-engineered Rhodobacter on sucrose, for hydrogen production (Sun, Q., Xiao, W., Xi, D., Shi, J., Yan, X. and Zhou, Z. (2010) Statistical Optimization of Biohydrogen Production from Sucrose by a Co-Culture of Clostridium acidisoli and Rhodobacter sphaeroides. International Journal of Hydrogen Energy, 35, 4076-4084). In another study by Yetis et al. (International Journal of Hydrogen Energy 25 (2000), p. 1035-1041), a doubling time of about 23 hours was reported for wild type Rhodobacter on sucrose, whereas the doubling time was about 3 to 6 hours on glucose. In light of the above, there is a strong need for engineered Rhodobacter microbial cell factories that are able to utilize sucrose, such as for terpene production.

The technical problem underlying the present invention shall be seen as the provision of means and methods complying with the aforementioned needs. The technical problem is solved by the embodiments characterized in the claims and herein below.

The present invention relates to Rhodobacter microbial cell factories genetically engineered to utilize sucrose as carbon source.

Specifically, the present invention pertains to a Rhodobacter cell engineered to utilize sucrose, comprising:
(i) a heterologous enzyme having invertase activity;
(ii) a heterologous enzyme having sucrose permease activity; and, optionally,
(iii) a heterologous enzyme having fructokinase activity.

In one embodiment of the Rhodobacter cell of the invention, said Rhodobacter cell engineered to utilize sucrose, comprises:
(i) a heterologous enzyme having invertase activity;
(ii) a heterologous enzyme having sucrose permease activity; and
(iii) a heterologous enzyme having fructokinase activity.

In a preferred embodiment of the Rhodobacter cell of the invention, said Rhodobacter cell comprises:
(i) a heterologous enzyme having invertase activity which comprises or consists of an amino acid sequence as shown in SEQ ID NO. 2, or an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 2;
(ii) a heterologous enzyme having sucrose permease activity which comprises or consists of an amino acid sequence as shown in SEQ ID NO. 6, or an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 6; and, optionally,
(iii) a heterologous enzyme having fructokinase activity which comprises or consists of an amino acid sequence as shown in SEQ ID NO. 4, or an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 4.

In another preferred embodiment of the Rhodobacter cell of the invention, said Rhodobacter cell comprises:
(i) a heterologous enzyme having invertase activity which comprises or consists of an amino acid sequence as shown in SEQ ID NO. 2, or an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 2;
(ii) a heterologous enzyme having sucrose permease activity which comprises or consists of an amino acid sequence as shown in SEQ ID NO. 6, or an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 6; and
(iii) a heterologous enzyme having fructokinase activity which comprises or consists of an amino acid sequence as shown in SEQ ID NO. 4, or an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 4.

In a further preferred embodiment of the Rhodobacter cell of the invention, said Rhodobacter cell comprises:
(i) a heterologous nucleic acid sequence as shown in SEQ ID No. 1, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 1, wherein said heterologous nucleic acid sequence encodes an enzyme having invertase activity as defined herein, preferably the invertase activity of the enzyme with the amino acid sequence shown in SEQ ID NO. 2;
(ii) a heterologous nucleic acid sequence as shown in SEQ ID No. 5, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 5, wherein said heterologous nucleic acid sequence encodes an enzyme having sucrose permease activity as defined herein, preferably the sucrose permease activity of the enzyme with the amino acid sequence shown in SEQ ID NO. 6; and, optionally,
(iii) a heterologous nucleic acid sequence as shown in SEQ ID No. 3, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 3, wherein said heterologous nucleic acid sequence encodes an enzyme having fructokinase activity as defined herein, preferably the fructokinase activity of the enzyme with the amino acid sequence shown in SEQ ID NO. 4.

In still another preferred embodiment of the Rhodobacter cell of the invention, said Rhodobacter cell comprises:
(i) a heterologous nucleic acid sequence as shown in SEQ ID No. 1, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 1, wherein said heterologous nucleic acid sequence encodes an enzyme having invertase activity as defined herein, preferably the invertase activity of the enzyme with the amino acid sequence shown in SEQ ID NO. 2;
(ii) a heterologous nucleic acid sequence as shown in SEQ ID No. 5, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 5, wherein said heterologous nucleic acid sequence encodes an enzyme having sucrose permease activity as defined herein, preferably the sucrose permease activity of the enzyme with the amino acid sequence shown in SEQ ID NO. 6; and
(iii) a heterologous nucleic acid sequence as shown in SEQ ID No. 3, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 3, wherein said heterologous nucleic acid sequence encodes an enzyme having fructokinase activity as defined herein, preferably the fructokinase activity of the enzyme with the amino acid sequence shown in SEQ ID NO. 4.

Preferably, the heterologous nucleic acid sequence of (i) and/or (ii) and/or (iii) above is codon-optimized for expression in Rhodobacter, preferably codon optimized for expression in Rhodobacter sphaeroides.

Preferably, the heterologous nucleic acid sequence of (i) and/or (ii) and/or (iii) above is under the control of a constitutive promoter.

In a further preferred embodiment of the Rhodobacter cell of the invention, said Rhodobacter cell comprises:
(i) a heterologous nucleic acid sequence as shown in SEQ ID No. 1, or a heterologous nucleic acid sequence having at least 70% sequence identity to SEQ ID No. 1, wherein said heterologous nucleic acid sequence encodes an enzyme having invertase activity of SEQ ID No. 2;
(ii) a heterologous nucleic acid sequence as shown in SEQ ID No. 5, or a heterologous nucleic acid sequence having at least 70% sequence identity to SEQ ID No. 5, wherein said heterologous nucleic acid sequence encodes an enzyme having sucrose permease activity of SEQ ID No. 6; and, optionally,
(iii) a heterologous nucleic acid sequence as shown in SEQ ID No. 3, or a heterologous nucleic acid sequence having at least 70% sequence identity to SEQ ID No. 3, wherein said heterologous nucleic acid sequence encodes an enzyme having fructokinase activity of SEQ ID No. 4.

In yet another preferred embodiment of the Rhodobacter cell of the invention, said Rhodobacter cell comprises:
(i) a heterologous nucleic acid sequence as shown in SEQ ID No. 1, or a heterologous nucleic acid sequence having at least 70% sequence identity to SEQ ID No. 1, wherein said heterologous nucleic acid sequence encodes an enzyme having invertase activity of SEQ ID No. 2;
(ii) a heterologous nucleic acid sequence as shown in SEQ ID No. 5, or a heterologous nucleic acid sequence having at least 70% sequence identity to SEQ ID No. 5, wherein said heterologous nucleic acid sequence encodes an enzyme having sucrose permease activity of SEQ ID No. 6; and
(iii) a heterologous nucleic acid sequence as shown in SEQ ID No. 3, or a heterologous nucleic acid sequence having at least 70% sequence identity to SEQ ID No. 3, wherein said heterologous nucleic acid sequence encodes an enzyme having fructokinase activity of SEQ ID No. 4.

Preferably, the heterologous nucleic acid sequence of (i) and/or (ii) and/or (iii) above is codon-optimized for expression in Rhodobacter, preferably codon optimized for expression in Rhodobacter sphaeroides.

Preferably, the heterologous nucleic acid sequence of (i) and/or (ii) and/or (iii) above is under the control of a constitutive promoter.

Preferably, the constitutive promoter driving the expression of the heterologous nucleic acid sequence encoding the enzyme having invertase activity and the optional heterologous nucleic acid sequence encoding the enzyme having fructokinase activity is the Pppa promoter, or the PcrtE promoter (see, e.g., US20200010822A1 and WO2018160066A1) and the constitutive promoter driving the expression of the heterologous nucleic acid sequence encoding the enzyme having sucrose permease activity is the Prplm promoter, in the Rhodobacter cell of the invention.

More preferably, the Pppa promoter comprises a sequence as shown in SEQ ID No. 7, and the Prplm promoter comprises a sequence as shown in SEQ ID No. 9.

In another preferred embodiment of the Rhodobacter cell of the invention, the enzyme having invertase activity comprises or consists of an amino acid sequence as shown in SEQ ID NO. 2 corresponding to the invertase CscA from E. coli KO11, the optional enzyme having fructokinase activity comprises or consists of an amino acid sequence as shown in SEQ ID NO. 4 corresponding to the fructokinase CscK from E. coli KO11, and the enzyme having sucrose permease activity comprises or consists of an amino acid sequence as shown in SEQ ID NO. 6 corresponding to the sucrose permease/proton symporter CscB from E. coli KO11.

The invention also relates to a method for producing a Rhodobacter cell engineered to utilize sucrose, comprising transforming a Rhodobacter cell, preferably a Rhodobacter sphaeroides cell, more preferably a cell of Cereibacter sphaeroides (Rhodobacter sphaeroides) ATCC35053 strain, with a vector comprising:
(i) a heterologous nucleic acid sequence as shown in SEQ ID No. 1, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 1, wherein said heterologous nucleic acid sequence encodes an enzyme having invertase activity as defined herein, preferably the invertase activity of the enzyme with the amino acid sequence shown in SEQ ID NO. 2;
(ii) a heterologous nucleic acid sequence as shown in SEQ ID No. 5, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 5, wherein said heterologous nucleic acid sequence encodes an enzyme having sucrose permease activity as defined herein, preferably the sucrose permease activity of the enzyme with the amino acid sequence shown in SEQ ID NO. 6; and, optionally,
(iii) a heterologous nucleic acid sequence as shown in SEQ ID No. 3, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 3, wherein said heterologous nucleic acid sequence encodes an enzyme having fructokinase activity as defined herein, preferably the fructokinase activity of the enzyme with the amino acid sequence shown in SEQ ID NO. 4.

In one preferred embodiment of this method of the invention for producing a Rhodobacter cell engineered to utilize sucrose, said method comprises transforming a Rhodobacter cell, preferably a Rhodobacter sphaeroides cell, more preferably a cell of Cereibacter sphaeroides (Rhodobacter sphaeroides) ATCC35053 strain, with a vector comprising:
(i) a heterologous nucleic acid sequence as shown in SEQ ID No. 1, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 1, wherein said heterologous nucleic acid sequence encodes an enzyme having invertase activity as defined herein, preferably the invertase activity of the enzyme with the amino acid sequence shown in SEQ ID NO. 2;
(ii) a heterologous nucleic acid sequence as shown in SEQ ID No. 5, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 5, wherein said heterologous nucleic acid sequence encodes an enzyme having sucrose permease activity as defined herein, preferably the sucrose permease activity of the enzyme with the amino acid sequence shown in SEQ ID NO. 6; and
(iii) a heterologous nucleic acid sequence as shown in SEQ ID No. 3, or a heterologous nucleic acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID No. 3, wherein said heterologous nucleic acid sequence encodes an enzyme having fructokinase activity as defined herein, preferably the fructokinase activity of the enzyme with the amino acid sequence shown in SEQ ID NO. 4.

Transforming methods and transfer of vectors or plasmids by conjugation of bacteria are standard procedures in the art and, thus, well described in the literature and, also, in the Examples; see, e.g., EP2875136, WO 2011/074954, US 9,260,709 and the publications cited in the introductory part.

Sequence identity or similarity is defined herein as a relationship between two or more polypeptide sequences or two or more nucleic acid sequences, as determined by comparing those sequences. Usually, sequence identities or similarities are compared over the whole length of the sequences, but may, however, also be compared only for a part of the sequences aligning with each other. In the art, "identity" or "similarity" also means the degree of sequence relatedness between polypeptide sequences or nucleic acid sequences, as the case may be, as determined by the match between such sequences. Sequence identity as used herein is preferably the value as determined by the EMBOSS Pairwise Alignment Algorithm "Needle", for instance at the server of the European Bioinformatics Institute (http://www.ebi.ac.uk/Tools/emboss/align/). For alignment of amino acid sequences the default parameters are: Matrix = Blosum62; Open Gap Penalty = 10.0; Gap Extension Penalty = 0.5. For alignment of nucleic acid sequences the default parameters are: Matrix = DNAfull; Open Gap Penalty = 10.0; Gap Extension Penalty = 0.5.

In this work, Rhodobacter microbial cell factories have been engineered to utilize sucrose for the first time, according to the best knowledge of the inventors. Engineered Rhodobacter microbial cell factories were shown to be able to use sucrose for growth. Surprisingly, the Rhodobacter cultures grow at a higher growth rate when using sucrose as a carbon source, compared to glucose. Next, terpene production was tested using sucrose as carbon source. Unexpectedly, the terpene production was increased compared to production on glucose as carbon source. Besides pure sucrose, also complex sucrose streams such as thick juice (concentrated sugarbeet extract) and molasse can be used for the Rhodobacter cell of the invention and result in increased growth rate and higher terpene titers compared to using glucose as carbon source, too. Sugarbeet streams have been used as sucrose source, in the present study.

As also shown in the following Examples, the three-enzyme sucrose utilisation pathway from E. coli KO11 has been optimized for expression in Rhodobacter. The sucrose permease, fructokinase and invertase genes have been codon optimized for expression in Rhodobacter. The invertase and fructokinase genes were organized in a synthetic operon under the control of a constitutive promoter, Pppa or PcrtE. Additionally, the RBS site between the genes was added to the synthetic operon for optimal expression of the fructokinase gene. The sucrose permease expression was driven by the Prplm constitutive promoter. The designed operons were synthesized by Genscript.

Firstly, the growth of Rhodobacter cultures using sucrose as a sole carbon source was tested.

For this purpose, the sucrose operon was cloned into the pBBR plasmid and conjugated into the Rhodobacter strain ATCC35053. The growth rates were measured by growing Rhodobacter cultures in Sistroms minimal medium and assessing the growth during exponential phase. With this experiment, the inventors demonstrated that the growth rate using sucrose is significantly increased, compared to growth with glucose. This was also shown for less-refined sucrose sugar streams, molasses and thick juice. This result was surprising since, when the sucrose operon was transferred from E. coli W to E. coli K-12, the growth rates observed were significantly lower than on glucose (Carruthers et al., ACS Synth. Biol. 2020, 9, 12, 3311-3321). Similarly, when the sucrose operon was transferred from E. coli KO11 to E. coli SZ63 (derivative W3110), cells grew more slowly than with glucose but reached a higher final density (Shukla et al., loc. cit.).

Next, the sucrose operon was cloned into the pRK415 plasmid and was conjugated into the Rhodobacter strain ATCC35053 carrying a zingiberene synthase on the pBBR plasmid. The production of terpenes was tested in rich medium RS102, using a dodecane overlay. The terpene content was analyzed by GC-MS. This experiment demonstrated that production of terpenes using sucrose was significantly increased, compared to production with glucose. This was also shown for less-refined sucrose sugar streams, molasses and thick juice.

When evaluating which of the three genes, sucrose permease, fructokinase or invertase, is the most important for sucrose metabolism by Rhodobacter, the inventors showed that strains lacking invertase do not grow on sucrose, strains lacking sucrose permease show impaired growth while the overexpression of fructokinase is not essential for growth on sucrose. This has been demonstrated by measuring growth rates in minimal medium.

Advantageously, a fermentation process using sucrose as carbon source instead of glucose leads to a more cost-efficient terpene production. Some microbes can efficiently utilize sucrose as a carbon source, as set forth in the introductory part. However, Rhodobacter naturally metabolises sucrose very poorly. Therefore, the import and assimilation of sucrose has been engineered and sugars have been channeled into the native sugar-assimilation pathways to facilitate bacterial growth with this carbon source and to optimally channel the sugars to the biosynthetic pathway for high-value terpenes, by the inventors. This approach opens the possibility to use sucrose such as from sugarcane or sugarbeet, and also less refined sucrose sugar streams such as molasses and thick juice for terpene production, increasing the cost-efficiency and sustainability of the production process even further. In a broader sense, as the cost of the fermentation process decreases, production of terpenes for more markets becomes feasible (e.g. agrochemicals, home-care products, bio-materials (plastics)).

In particular, the Rhodobacter cell of the invention allows to utilize sucrose as an alternative sugar source which leads to higher growth rate and higher production then when using glucose.

In addition, improving the cost-efficiency of the fermentation makes it possible to not only target plant terpenes with very high value like flavours, but apply the approach to other less costly classes of molecules, such as UV scavengers, natural colorants and in natural crop protectants. This invention may also be applicable to production of other compounds then terpenes in Rhodobacter, for example coenzyme Q10.

The results and achievements by the inventors may be summarized as follows:
- Design and optimization of the sucrose operon for expression in Rhodobacter.
- Demonstration of increased growth rate of Rhodobacter cell factories utilising sucrose as carbon source.
- Demonstration of increased terpene production of Rhodobacter cell factories utilising sucrose as carbon source.
- Demonstration of increased growth and terpene production of Rhodobacter cell factories utilising less-refined sucrose sugar streams (molasses, thick juice).
- Demonstrating that the quality of the produced terpenes is equal then of those produced on glucose, as tested by GC-MS analysis.

D-Sucrose, a disaccharide of glucose and fructose, is common in the biosphere due to its production in many plant tissues. It is a major product of human agriculture and can be advantageous for microbially-mediated production of alcohols and other industrially-relevant chemicals (Peters S., Rose T., Moser M. (2010). Sucrose: a prospering and sustainable organic raw material. Top. Curr. Chem. 294, 1-23). However, the ability to utilize sucrose as a carbon and energy source is not universal among microbes, and is highly variable among Gram-negative bacteria of the family Enterobacteriaceae (Le Bouguénec C., Schouler C. (2011). Sugar metabolism, an additional virulence factor in enterobacteria. Int. J. Med. Microbiol. 301, 1-6). For example, isolates of Salmonella and Shigella rarely utilize sucrose, but isolates of Enterobacter and Klebsiella usually do.

Escherichia coli is a model prokaryote, an important pathogen, and a key organism for industrial biotechnology. As already mentioned, Escherichia coli W (ATCC 9637) grows particularly quickly on sucrose; see Archer et al. (2011; loc. cit.). The complete genome of E. coli W is shown in GenBank accession number CP002185.1. Genome sequencing indicated that sucrose is metabolized via the csc genes in this strain. The csc regulon was originally described in E. coli EC3132 (Jahreis K, Bentler L, Bockmann J, Hans S, Meyer A, Siepelmeyer J, Lengeler JW. 2002. Adaptation of sucrose metabolism in the Escherichia coli wild-type strain EC3132. J. Bacteriol. 184:5307-5316) and consists of four open reading frames which encode a transcriptional repressor (CscR), a sucrose hydrolase or invertase (CscA), a sucrose permease (CscB), and a fructokinase (CscK). The csc catabolic genes are negatively controlled by CscR, which presumably represses transcription in the absence of sucrose and at low sucrose concentrations (<2 g/liter). The csc operon from strain E. coli KO11 is similarly encoding a repressor protein (cscR), an operon encoding invertase (cscA), and a bicistronic operon (cscKB) encoding fructokinase and an anion symport for sucrose, respectively. The sucrose permease/proton symporter, CscB, transports sucrose into the cell. Intracellular sucrose is then hydrolysed to glucose and fructose by CscA. These two sugars are then phosphorylated into glucose-6-phosphate and fructose-6-phosphate by glucokinase (Glk) and CscK, respectively. The phosphorylated sugars are then assimilated into glycolysis.

The nucleic acid sequence encoding the invertase CscA (also known as sucrose-6-phosphate hydrolase, beta-fructofuranosidase, or sucrase)) from E.coli KO11 is shown in GenBank accession number AY314757. Said invertase CscA hydrolyses terminal non-reducing beta-D-fructo-furanoside residues in beta-D-fructofuranosides. It enables the bacterium to metabolize sucrose as a sole carbon source.

SEQ ID No. 1 shows the codon optimized nucleic acid sequence encoding the invertase CscA from E.coli KO11, i.e. said nucleic acid sequence has been optimized in terms of codon usage for expression in Cereibacter sphaeroides ((van Niel) Hördt et al.) (ATCC35053). Cereibacter sphaeroides strain 81-2 is an anaerobic bacterium that was isolated from a sewage settling pond in Indiana, United States. Its former name was Rhodobacter sphaeroides (van Niel) Imhoff et al. Cereibacter sphaeroides and Rhodobacter sphaeroides are used interchangeably herein. The amino acid sequence of the invertase CscA from E. coli KO11 is depicted in SEQ ID No. 2 or also in UniProt P40714.

Invertase activity can be assayed by methods described in the art (see, e.g. Sabri et al., Appl Environ Microbiol. 2013 Jan; 79(2): 478-487; Schmid K, Schupfner M, Schmitt R. 1982. Plasmid-mediated uptake and metabolism of sucrose by Escherichia coli K-12. J. Bacteriol. 151:68-76).

The nucleic acid sequence encoding the fructokinase CscK from E.coli KO11 is shown in GenBank accession number AY314757. Said fructokinase CscK converts fructose into fructose-6-phosphate.

SEQ ID No. 3 shows the codon optimized nucleic acid sequence encoding the fructokinase CscK from E.coli KO11, i.e. said nucleic acid sequence has been optimized in terms of codon usage for expression in Cereibacter sphaeroides ((van Niel) Hördt et al.). The amino acid sequence of the fructokinase CscK from E. coli KO11 is depicted in SEQ ID No. 4.

Fructokinase activity can be assayed by methods known in the art (see, e.g. Sabri et al., Appl Environ Microbiol. 2013 Jan; 79(2): 478-487; Sprenger GA, Lengeler JW. 1988. Analysis of sucrose catabolism in Klebsiella pneumoniae and in Scr+ derivatives of Escherichia coli K12. J. Gen. Microbiol. 134:1635-1644).

The nucleic acid sequence encoding the sucrose permease CscB (also known as sucrose transporter or sucrose permease/proton symporter) from E.coli KO11 is shown in GenBank accession number AY314757. The sucrose permease/proton symporter, CscB, transports sucrose into the bacterial cell.

The activity of the sucrose permease CscB can be assayed by methods known in the art (see, e.g., M Sahin-Tóth et al., Biochemistry. 2000 May 23;39(20):6164-9).

SEQ ID No. 5 shows the codon optimized nucleic acid sequence encoding the sucrose permease CscB from E.coli KO11, i.e. said nucleic acid sequence has been optimized in terms of codon usage for expression in Cereibacter sphaeroides ((van Niel) Hördt et al.). The amino acid sequence of the sucrose permease CscB from E. coli KO11 is depicted in SEQ ID No. 6.

Assays for sugar analyses and the determination of concentrations of sugars, such as sucrose, glucose, or fructose, are well known in the art and include, for example, high-pressure liquid chromatography (HPLC). To this end, extracellular samples can be collected from shake flask fermentations by centrifugation, and the supernatant can be stored at -20°C for subsequent chromatographic analyses. Concentrations of sugars, such as sucrose, glucose, or fructose, can then be analyzed by high-pressure liquid chromatography (HPLC) using, e.g., an Agilent 1200 Series chromatograph (Agilent, Forest Hill, Victoria, Australia) as described in the literature (see, for instance, Sabri et al., Appl Environ Microbiol. 2013 Jan; 79(2): 478-487; Arifin Y, Sabri S, Sugiarto H, Krömer JO, Vickers CE, Nielsen LK. 2010. Deletion of cscR in Escherichia coli W improves growth and poly-3-hydroxybutyrate (PHB) production from sucrose in fed batch culture. J. Biotechnol. 156:275-278).

The Rhodobacter cell as referred to herein is preferably selected from the group of Rhodobacter capsulatus and Rhodobacter sphaeroides, more preferably Rhodobacter sphaeroides. Even more preferably, the Rhodobacter cell is from Rhodobacter sphaeroides strain ATCC35053.

Rhodobacter sphaeroides is an example of a microbial organism naturally containing all genes needed for expressing enzymes catalyzing the various reaction steps in the DXP pathway, enabling the intracellular production of IPP and DMAPP.

In another preferred embodiment of the Rhodobacter cell of the invention, the Rhodobacter cell exhibits (i) an increased growth rate on sucrose as carbon source, compared to the growth rate on glucose as carbon source, and/or (ii) improved sucrose utilization compared to a wildtype Rhodobacter cell.

The measurements in the Examples show that the Rhodobacter sphaeroides strain ATCC35053 shows very poor growth when sucrose is given as a carbon source, with observed growth rates of 0.03 h-1, compared to 0.19 h-1 for glucose as carbon source. When sucrose was provided as carbon source to the engineered strain Cs_pBBR-IFT comprising the invertase gene, sucrose permease gene and the fructokinase gene, said engineered strain showed an improved growth rate of 0.19 h-1 compared to the growth rate of 0.16 h-1 of the same engineered strain on glucose.

An improved growth rate of the Rhodobacter cell of the invention compared to a wildtype Rhodobacter cell, such as a cell of the Rhodobacter sphaeroides ATCC35053 strain, means an at least 1.5 fold, at least 2 fold, at least 3 fold, at least 4 fold, at least 5 fold, at least 6 fold or at least 6.5 fold increase of the growth rate on sucrose. Preferably, said increase is between 5 fold and 7 fold, more preferably between 6 fold and 6.5 fold.

An increased growth rate on sucrose as carbon source, compared to the growth rate on glucose as carbon source, of the Rhodobacter cell of the invention means an at least 1.1 fold, at least 1.2 fold, at least 1.3 fold, at least 1.4 fold, or at least 1.5 fold increased growth rate of the Rhodobacter cell of the invention on sucrose, compared to glucose. Preferably, said increase is between 1.1 fold and 1.3 fold.

Preferably, the growth rate of the Rhodobacter cell of the invention on sucrose is between 0.10 h⁻¹ and 0.20 h⁻¹, preferably between 0.15 h⁻¹ and 0.20 h⁻¹.

An improved sucrose utilization of the Rhodobacter cell of the invention compared to a wildtype Rhodobacter cell such as Rhodobacter sphaeroides ATCC35053 means that the Rhodobacter cell of the invention has a growth rate which is at least equal to or higher than 50% of the growth rate on glucose. Alternatively, an improved sucrose utilization of the Rhodobacter cell of the invention compared to a wildtype Rhodobacter cell such as Rhodobacter sphaeroides ATCC35053 means that the Rhodobacter cell of the invention has a doubling time that is equal to or less than 2-fold of the doubling time on glucose.

Growth curve measurement based on optical density (OD) is one of the most commonly used methods in microbiology for monitoring the growth and proliferation of microbes in time, which provides a simple, reliable and routine way to understand various aspects of the microbes. Bacterial growth can also be evaluated with microplate readers; see, e.g., Krishnamurthi et al., PLoS One. 2021; 16(1): e0245205. Thus, growth rates of bacteria can be determined by methods known in the art and as shown in the Examples.

Assays for measuring sucrose or glucose utilization are set forth elsewhere herein and shown in the Examples.

Preferably, sucrose is used as the sole carbon source, for the Rhodobacter cell of the invention, or in the use or method of the invention.

Preferably, sucrose is refined sucrose, preferably from sugarcane or sugarbeet, less-refined sucrose sugar streams, molasses, thick juice or sugarcane juice.

As set forth above, the Rhodobacter cell of the invention can be advantageously used for terpene production using sucrose as carbon source. The major terpenoid biosynthetic pathways may be summarized as follows. All terpenoids are derived from two isomeric 5-carbon precursors, isopentenyl diphosphate (IPP), and dimethylallyl diphosphate (DMAPP). In turn, IPP and DMAPP are formed via two pathways, the cytosolic mevalonate (MVA) pathway originating from acetyl-CoA and the pyruvate and glyceraldehyde-3-phosphate (G3P)-derived 2-C-methyl-D-erythritol-4-phosphate (MEP) pathway located in the plastids. However, active transfers of IPP, DMAPP, GPP, and FPP across the plastidial membrane enable some degree of pathway crosstalk. In addition, interconversion of IPP and DMAPP with their respective monophosphate forms IP and DMAP by IP kinase (IPK) and Nudix hydrolase enzymes can impact pathway flux in terpenoid metabolism. Except for isoprene and hemiterpene (C5) biosynthesis, condensation of IPP and DMAPP units generates prenyl diphosphate intermediates of different chain length. Condensation of IPP and DMAPP yields geranyl diphosphate (GPP) as the precursor to monoterpenoids (C10), fusing GPP with an additional IPP affords the sesquiterpenoid (C15) precursor farnesyl diphosphate (FPP), and fusing FPP with IPP generates geranylgeranyl diphosphate (GGPP) *en route* to diterpenoids (C20). Furthermore, condensation of two FPP or two GGPP molecules forms the central substrates of triterpenoid (C30) and carotenoids (C40), respectively. Terpene synthases (TPS) are key gatekeepers in the biosynthesis of C10-C20 terpenoids, catalyzing the committed scaffold-forming conversion of the respective prenyl diphosphate substrates into a range of hydrocarbon or oxygenated structures. These TPS products can then undergo various oxygenations through the activity of cytochrome P450 monooxygen-ases (P450), followed by further possible functional decorations.

Terpenoids compose the biggest and most diversified class of chemical substances discovered in plants, encompassing over 40,000 individual compounds. The evolutionary success of the terpenoid metabolites has largely depended on the flexibility of their building molecules of various sizes. Indeed, terpenoids, arising from the two basic five-carbon (C5) isoprenoid units, isopentenyl diphosphate, and its isomer, dimethylallyl diphosphate, can be categorized as hemi-terpenoids (C5), monoterpenoids (C10), sesquiterpenoids (C15), diterpenoids (C20), triterpenoid (C30), tetraterpenoid (C40), or polyterpenoids (C5n), based on the number of C5 units they contain (Tholl D., Lee S. Terpene specialized metabolism in Arabidopsis thaliana. Arab. Book. 2011 ;9:e0143).

The terms "terpene", "terpenoid" and "isoprenoid" are used interchangeably herein.

In a further preferred embodiment of the Rhodobacter cell of the invention, the Rhodobacter cell further comprises (i) heterologous nucleic acid sequences encoding enzymes for catalyzing the reaction steps of the mevalonate pathway or another metabolic pathway such as the deoxyxylu-lose-5-phosphate (DXP) pathway enabling the production of C5 prenyl diphosphates isopentenyl diphosphate (IPP) and dimethylallyl diphosphate (DMAPP) as isoprenoid building blocks and (ii) ) heterologous nucleic acid sequences encoding enzymes for having prenyl transferase activity catalyzing the head-to-tail condensation of the C5 prenyl diphosphates producing longer prenyl diphosphates, such as the sesquiterpene precursor farnesyl diphosphate (FPP).

The Rhodobacter cell of the invention can be used for the production of a monoterpene.

To this end, in one embodiment of the Rhodobacter cell of the invention, the Rhodobacter cell of the invention further comprises:
(i) nucleic acid sequences, preferably heterologous nucleic acid sequences, encoding enzymes of a mevalonate pathway for making isoprenyl pyrophosphate (IPP) and its isomer dimethylallyl pyrophosphate (DMAPP),
(ii) a nucleic acid sequence, preferably a heterologous nucleic acid sequence, encoding an enzyme having catalytic activity for the condensation of IPP and DMAPP into geranyl diphosphate (GPP), and
(iii) a nucleic acid sequence, preferably a heterologous nucleic acid sequence, encoding an enzyme having monoterpene synthase activity in the conversion of GPP into a monoterpene.

The Rhodobacter cell of the invention which is able to use sucrose as carbon source can be further genetically modified or engineered by incorporation of a gene that is coding for a protein having terpenoid synthase activity, such as a monoterpene synthase or a sesquiterpene synthase, in addition to genes encoding enzymes for production of IPP, either via the mevalonate pathway or the DXP pathway. These pathways are known in the art, and have been described, e.g., by Withers & Keasling in Appl. Microbiol. Biotechnol. (2007) 73: 980-990. In particular, Figure 1 of said publication shows the enzymes that play a role in the mevalonate pathway; see also, e.g., EP2875136 for a detailed description of enzymes required for the mevalonate pathway and nucleic acid sequences encoding these enzymes. The disclosure content of the publication by Withers & Keasling in Appl. Microbiol. Biotechnol. (2007) 73: 980-990, EP2875136, and WO 2019/045568 A1 (Isobionics B.V.) are incorporated herewith by reference. The genes of these pathways may each independently be homologous or heterologous to the Rhodobacter cell of the invention.

It is preferred that the enzyme having monoterpene synthase activity is selected from the group of enzymes having beta-pinene synthase activity, alpha-pinene synthase activity, myrcene synthase activity, limonene synthase activity, linalool synthase activity, sabinene synthase activity, bisabolene synthase activity, and geraniol synthase activity.

Preferably, the enzymes of the mevalonate pathway comprise:
(i) a heterologous enzyme having catalytic activity in the reaction of acetoacyl-CoA with acetyl-CoA to form HMG-CoA;
(ii) a heterologous enzyme having catalytic activity in the conversion of HMG-CoA to mevalonate;
(iii) a heterologous enzyme having catalytic activity in the phosphorylation of mevalonate to mevalonate 5-phosphate;
(iv) a heterologous enzyme having catalytic activity in the conversion of mevalonate 5- phosphate to mevalonate 5-pyrophosphate;
(v) a heterologous enzyme having catalytic activity in the conversion of mevalonate 5- pyrophosphate to IPP; and
(vi) a heterologous or homologous enzyme having catalytic activity in the reversible conversion of IPP to DMAPP,
   in the Rhodobacter cell of the invention.

Preferably, the Rhodobacter cell of the invention is free of genes encoding a heterologous enzyme having catalytic activity in the reaction of conversion of two molecules of acetyl-CoA to acetoacyl-CoA.

It is further preferred that the monoterpene is selected from the group consisting of beta-pinene, myrcene, alpha-pinene, limonene, linalool, sabinene, bisabolene and geraniol.

The Rhodobacter cell of the invention can also be used for the production of a sesquiterpene.

To this end, in another preferred embodiment of the Rhodobacter cell of the invention, the Rhodobacter cell of the invention further comprises:
(i) nucleic acid sequences, preferably heterologous nucleic acid sequences, encoding enzymes of a mevalonate pathway for making isoprenyl pyrophosphate (IPP);
(ii) a nucleic acid sequence, preferably a heterologous nucleic acid sequence, encoding an enzyme having catalytic activity in the conversion of IPP into farnesyl pyrophosphate (FPP); and
(iii) a nucleic acid sequence, preferably a heterologous nucleic acid sequence, encoding an enzyme having sesquiterpene synthase activity in the conversion of FPP into a sesquiterpene, wherein the host cell is free of heterologous enzymes having catalytic activity in the reaction of conversion of two molecules of acetyl-CoA to acetoacyl-CoA.

Preferably, the enzymes of the mevalonate pathway comprise:
(i) a heterologous enzyme having catalytic activity in the reaction of acetoacyl-CoA with acetyl-CoA to form HMG-CoA;
(ii) a heterolgous enzyme having catalytic activity in the conversion of HMG-CoA to mevalonate;
(iii) a heterologous enzyme having catalytic activity in the phosphorylation of mevalonate to mevalonate 5-phosphate;
(iv) a heterologous enzyme having catalytic activity in the conversion of mevalonate 5- phosphate to mevalonate 5-pyrophosphate;
(v) a heterologous enzyme having catalytic activity in the conversion of mevalonate 5- pyrophosphate to IPP; and
(vi) a heterologous or homologous enzyme having catalytic activity in the reversible conversion of IPP to DMAPP,
in the Rhodobacter cell of the invention.

It is preferred that the enzyme having sesquiterpene synthase activity is selected from the group of enzymes having valencene synthase activity, bisabolene synthase activity, bisabolol synthase activity, bergamotene synthase activity, farnesene synthase activity, zizaene synthase activity, santalene synthase activity, zingiberene synthase activity and patchoulol synthase activity.

It is further preferred that the sesquiterpene is valencene, bisabolene, bisabolol, bergamotene, farnesene, zizaene, santalene zingiberene, or patchoulol.

Preferably, the at least one heterologous nucleic acid sequence encoding the zingiberene synthase activity is shown in SEQ ID NO. 10 which corresponds to the nucleic acid sequence encoding the zingiberene synthase from Callitropsis nootkatensis - nootka cypress. Preferably, said nucleic acid sequence is codon-optimized for expression in Rhodobacter. SEQ ID NO. 11 depicts the amino acid sequence of the zingiberene synthase from Callitropsis nootkatensis - nootka cypress.

Preferably, the Rhodobacter cell exhibits an increased production of a sesquiterpene, preferably zingiberene, using sucrose as carbon source, preferably as sole carbon source, compared to the production of said sesquiterpene, preferably zingiberene, using glucose as carbon source, preferably as sole carbon source.

An increased production of a sesquiterpene, preferably zingiberene, using sucrose as carbon source, in comparison to glucose as carbon source, in the Rhodobacter cell of the invention means an increase of at least 10%, at least 20%, at least 30%, or at least 35% of the concentration of sesquiterpene (g/l), preferably of the amount of zingiberene. Preferably, said increase is between 20% and 50%, more preferably between 30% and 40%, even more preferably between 35% and 38%.

The Rhodobacter cell of the invention can also be used for the production of a diterpene, such as sclareol.

Thus, in another preferred embodiment of the Rhodobacter cell of the invention, said Rhodobacter cell further comprises:
(i) a nucleic acid sequence encoding an enzyme having geranylgeranyl pyrophosphate synthase activity;
(ii) a nucleic acid sequence encoding an enzyme having labda-13-en-8-ol diphosphate synthase activity; and
(iii) a nucleic acid sequence encoding an enzyme having sclareol synthase activity.

Preferably, said Rhodobacter cell exhibits an increased production of sclareol. An increased production of sclareol, using sucrose as carbon source, in comparison to glucose as carbon source, in the Rhodobacter cell of the invention means an increase of at least 10%, at least 20%, at least 30%, or at least 35% of the concentration of sclareol (g/l). Preferably, said increase is between 10% and 50%, more preferably between 20% and 40%.

Sclareol biosynthesis and enzymes required for the production of sclareol are known in the art; see, e.g., Caniard et al., BMC Plant Biol. 2012 Jul 26:12:119; Ma et al., Plant Science, Volume 304, March 2021, 110790; Camille Chalvin, Thèse de doctorat de l'Université Paris-Saclay préparée à l'Université Paris-Sud; École doctorale n°567 Sciences du Végétal: du gène à l'écosystème (SDV); Spécialité de doctorat: Biologie Sclareol biosynthesis in clary sage and its regulation (2019); US 9,725,740; and WO2023280677.

The invention also relates to a method for preparing a monoterpene, a sesquiterpene, a diterpene, a triterpene, a tetraterpene, a flavour, an aroma compound, an UV scavenger, a natural colorant, a natural crop protectant or an isoprenoid moiety containing compound such as coenzyme Q10, comprising culturing a Rhodobacter cell according to the invention in a culture medium comprising sucrose as carbon source, preferably sole carbon source, for the monoterpene, sesquiterpene, diterpene, triterpene, tetraterpene, flavour, aroma compound, UV scavenger, natural colorant, natural crop protectant or the isoprenoid moiety containing compound such as coenzyme Q10.

The invention further pertains to the use of the Rhodobacter cell of the invention for the production of a monoterpene, a sesquiterpene, a diterpene, a triterpene, a tetraterpene, a flavour, an aroma compound, an UV scavenger, a natural colorant, a natural crop protectant or for production of an isoprenoid moiety containing compound such as coenzyme Q10, preferably utilizing sucrose as carbon source, more preferably utilizing sucrose as sole carbon source.

### Definitions and further embodiments

The term "or" as used herein is defined as "and/or" unless specified otherwise. The term "a" or "an" as used herein is defined as "at least one" unless specified otherwise. When referring to a noun (e.g. a compound, an additive, etc.) in the singular, the plural is meant to be included.

The term "recombinant" in relation to a recombinant cell, vector, nucleic acid sequence or the like as used herein, refers to a cell, vector, nucleic acid or the like, containing a nucleic acid sequence not naturally occurring in that cell, vector, nucleic acid or the like and/or not naturally occurring at that same location. Generally, said nucleic acid sequence has been introduced into that strain (cell) using recombinant DNA techniques.

The term "heterologous" when used with respect to a nucleic acid sequence (DNA or RNA) or amino acid sequence, protein or enzyme, refers to a nucleic acid sequence or amino acid sequence, protein or enzyme that does not occur naturally as part of the organism, cell, genome or DNA or RNA sequence in which it is present, or that is found in a cell or location or locations in the genome or DNA or RNA sequence that differ from that in which it is found in nature. Heterologous nucleic acid sequences or amino acid sequences, proteins or enzmyes are not endogenous to the cell into which they are introduced, but have been obtained from another cell or synthetically or recombinantly produced. To provide an example, the Rhodobacter cell of the invention comprises, inter alia, a heterologous nucleic acid sequence as shown in SEQ ID No. 1. This sequence corresponds to a nucleic acid sequence encoding the invertase cscA from E. coli KO11 which has been optimized in terms of codon usage for expression in Rhodobacter sphaeroides. Generally, though not necessarily, such heterologous nucleic acid sequences encode proteins or enzymes that are not normally produced by the cell in which the DNA or RNA is expressed. A gene that is endogenous to a particular host cell but has been modified from its natural form, through, for example, the use of DNA shuffling, is also called heterologous. The term "heterologous" also includes non-naturally occurring multiple copies of a naturally occurring DNA sequence. Thus, the term "heterologous" may refer to a DNA segment that is foreign or heterologous to the cell, or homologous to the cell but in a position and/or a number within the host cell nucleic acid in which the segment is not ordinarily found. Exogenous DNA segments are expressed to yield exogenous polypeptides. A "homologous" DNA sequence is a DNA sequence that is naturally associated with a host cell into which it is introduced. Any nucleic acid sequence or amino acid sequence that one of skill in the art would recognize as heterologous or foreign to the cell in which it is expressed is herein encompassed by the term heterologous nucleic acid or protein.

The term "genetic engineering" or "genetically engineered" or briefly "engineered" means the artificial manipulation, modification, and recombination of DNA or other nucleic acid molecules in order to modify an organism or population of organisms. The term "genetic engineering" is generally used to refer to methods of recombinant DNA technology. The terms "genetically engineered", "engineered" and "modified" are used interchangeably herein.

The terms "modified", "modification", "mutated", "mutation", or "variant" as used herein regarding a protein or polypeptide or enzyme compared to another protein or peptide or enzyme (in particular compared to the polypeptide, protein or enzyme comprising or consisting of amino acids in the sequences shown herein), is used to indicate that the modified protein or polypeptide or enzyme has at least one difference in the amino acid sequence compared to the protein or polypeptide or enzyme with which it is compared, e.g. a wildtype protein, polypeptide, or enzyme. The terms are used irrespective of whether the modified/mutated protein or enzyme actually has been obtained by mutagenesis of nucleic acid sequences encoding these amino acids or modification of the polypeptide/protein/enzyme or in another manner, e.g. using artificial gene-synthesis methodology. Mutagenesis is a well-known method in the art, and includes, for example, site-directed mutagenesis by means of PCR or via oligonucleotide- mediated mutagenesis as described in Sambrook, J., and Russell, D.W. Molecular Cloning: A Laboratory Manual.3d ed., Cold Spring Harbor Laboratory Press, Cold Spring Harbor, NY, (2001). The term "modified", "modification", "mutated", "mutation" or "variant" as used herein regarding genes or nucleic acid sequences is used to indicate that at least one nucleotide in the nucleotide sequence of that gene or a regulatory sequence thereof, is different from the nucleotide sequence that it is compared with, e.g. a wildtype nucleotide sequence. The terms are used irrespective of whether the modified/mutated nucleotide sequence actually has been obtained by mutagenesis. A modification/mutation may in a particular be a replacement of an amino acid respectively nucleotide by a different one, a deletion of an amino acid respectively nucleotide or an insertion of an amino acid respectively nucleotide.

The term "codon optimized" as used herein means a nucleic acid sequence which is optimized in terms of codon usage for expression in Cereibacter sphaeroides ((van Niel) Hördt et al.) (former name Rhodobacter sphaeroides (van Niel) Imhoff et al.). The term "codon-optimized nucleic acid sequence" as used herein denotes a nucleotide sequence which has been converted to a sequence having an identical translated sequence but with alternative codon usage, for expression in Rhodobacter strains, as defined by Lathe, 1985, J. Mol. Biol. 183: p. 1-12. Gene or codon-optimization takes advantage of the degeneracy of the genetic code. Because of degeneracy, one protein can be encoded by many alternative nucleic acid sequences. Codon preference (codon usage bias) differs in each organism, and it can create challenges for expressing recombinant proteins in heterologous expression systems, resulting in low and unreliable expression.

The methodology of codon-optimization for expression in Rhodobacter cells may be summarized as follows:
(i) Identify placement of codons for proper open reading frame.
(ii) Compare wild type codon for observed frequency of use by Rhodobacter genes.
(iii) If codon is not the most commonly employed, replace it with an optimal codon for high expression in Rhodobacter cells.
(iv) Repeat this procedure until the entire gene segment has been replaced.
(v) Inspect new gene sequence for undesired sequences generated by these codon replacements (e.g. unwanted restriction sites, etc.) and substitute codons that eliminate these sequences.
(vi) Assemble synthetic gene segments and test for improved expression.

Methods and tools for codon-optimization of sequences are well described in the art; see, e.g., US Patent 8,326,547; Ternette N., et al., Virology Journal 2007, Expression of RNA virus proteins by RNA polymerase II dependent expression plasmids is hindered at multiple steps. https://doi.org/10.1186/1743-422X-4-51; Haas J, Park EC, Seed B: Codon usage limitation in the expression of HIV-1 envelope glycoprotein. Curr Biol 1996, 6: 315-324. 10.1016/S0960-9822(02)00482-7; Wagner R, Graf M, Bieler K, Wolf H, Grunwald T, Foley P, Uberla K: Rev-independent expression of synthetic gag-pol genes of human immunodeficiency virus type 1 and simian immunodeficiency virus: implications for the safety of lentiviral vectors. Hum Gene Ther 2000, 11: 2403-2413. 10.1089/104303400750038507; or Morton CJ, Cameron R, Lawrence LJ, Lin B, Lowe M, Luttick A, Mason A, Kimm-Breschkin J, Parker MW, Ryan J, Smout M, Sullivan J, Tucker SP, Young PR: Structural characterization of respiratory syncytial virus fusion inhibitor escape mutants: homology model of the F protein and a syncytium formation assay. Virology 2003, 311: 275-288. 10.1016/S0042-6822(03)00115-6. Further, use may be made of codon optimization or codon pair optimization, e.g. based on a method as described in WO 2008/000632, or as offered by commercial DNA synthesizing companies like DNA2.0, Geneart, and GenScript. Examples of a codon optimized sequence include SEQ ID NO: 1, 3 or 5.

As appreciated by those skilled in the art, the use of alternative codons encoding the same protein sequence removes the constraints on expression E. coli KO11 nucleic acid sequences by Rhodobacter cells.

The terms "open reading frame" and "ORF" refer to a sequence of adjacent codons that starts with a start codon, followed by a series of codons for amino acids, and ends with a stop codon. This sequence has the potential to be translated into a polypeptide/protein product.

The term "gene" is used broadly to refer to any segment of nucleic acid associated with a biological function. Thus, genes include coding sequences and/or the regulatory sequences required for their expression. For example, gene refers to a nucleic acid fragment that expresses mRNA or functional RNA, or encodes a specific protein, and which includes regulatory sequences. Genes also include non-expressed DNA segments that, for example, form recognition sequences for other proteins. Genes can be obtained from a variety of sources, including cloning from a source of interest or synthesizing from known or predicted sequence information, and may include sequences designed to have desired parameters. The term "chimeric gene" refers to any gene that contains 1) DNA sequences, including regulatory and coding sequences that are not found together in nature, or 2) sequences encoding parts of proteins not naturally adjoined, or 3) parts of promoters that are not naturally adjoined. Accordingly, a chimeric gene may comprise regulatory sequences and coding sequences that are derived from different sources, or comprise regulatory sequences and coding sequences derived from the same source, but arranged in a manner different from that found in nature.

The term "transgenic" for a transgenic cell or organism as used herein, refers to an organism or cell (which cell may be an organism per se or a cell of a multi-cellular organism from which it has been isolated) containing a nucleic acid sequence not naturally occurring in that organism or cell and which nucleic acid sequence has been introduced into that organism or cell (i.e. has been introduced in the organism or cell itself or in an ancestor of the organism or an ancestral organism of an organism of which the cell has been isolated) using recombinant DNA techniques.

A "transgene" refers to a gene that has been introduced into the genome by transformation and preferably is stably maintained. Transgenes may include, for example, genes that are either heterologous or homologous to the genes of a particular cell/organism to be transformed. Additionally, transgenes may comprise native genes inserted into a non-native organism, or chimeric genes. The term "endogenous gene" refers to a native gene in its natural location in the genome of an organism. A "foreign" gene refers to a gene not normally found in the host organism but that is introduced by gene transfer.

"Transformation" and "transforming", as used herein, refers to the introduction of a heterologous nucleotide sequence into a host cell, irrespective of the method used for the insertion, for example, direct uptake, transduction, conjugation, f-mating or electroporation. The exogenous polynucleotide may be maintained as a non-integrated vector, for example, a plasmid, or alternatively, may be integrated into the host cell genome.

"Coding sequence" refers to a DNA or RNA sequence that codes for a specific amino acid sequence and excludes the non-coding sequences. It may constitute an "uninterrupted coding sequence", i.e. lacking an intron, such as in a cDNA or it may include one or more introns bound by appropriate splice junctions. An "intron" is a sequence of RNA which is contained in the primary transcript but which is removed through cleavage and re-ligation of the RNA within the cell to create the mature mRNA that can be translated into a protein.

"Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences include enhancers, promoters, translation leader sequences, introns, and polyadenylation signal sequences. They include natural and synthetic sequences as well as sequences which may be a combination of synthetic and natural sequences. As is noted above, the term "suitable regulatory sequences" is not limited to promoters. Examples of regulatory sequences include promoters (such as transcriptional promoters, constitutive promoters, inducible promoters), operators, enhancers, mRNA ribosomal binding sites, and appropriate sequences which control transcription and translation initiation and termination. Nucleic acid sequences are "operably linked" when the regulatory sequence functionally relates to the DNA or cDNA sequence. As used herein, the term "operably linked" or "operatively linked" refers to a juxtaposition wherein the components so described are in a relationship permitting them to function in their intended manner. A control sequence "operably linked" to another control sequence and/or to a coding sequence is ligated in such a way that transcription and/or expression of the coding sequence is achieved under conditions compatible with the control sequence. Generally, operably linked means that the nucleic acid sequences being linked are contiguous and, where necessary to join two protein coding regions, contiguous and in the same reading frame. Each of the regulatory sequences may independently be selected from heterologous and homologous regulatory sequences.

"Promoter" refers to a nucleotide sequence, usually upstream (5') to its coding sequence, which controls the expression of said coding sequence by providing the recognition for RNA polymerase and other factors required for proper transcription. "Promoter" includes a minimal promoter that is a short DNA sequence comprised of a TATA box and other sequences that serve to specify the site of transcription initiation, to which regulatory elements are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers.

Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements derived from different promoters found in nature, or even be comprised of synthetic DNA segments. A promoter may also contain DNA sequences that are involved in the binding of protein factors which control the effectiveness of transcription initiation in response to physiological or developmental conditions.

The term "nucleic acid sequence" as used herein, includes reference to a deoxyribonucleotide or ribonucleotide polymer, i.e. a polynucleotide, in either single-or double-stranded form, and unless otherwise limited, encompasses known analogues having the essential nature of natural nucleotides in that they hybridize to single-stranded nucleic acid sequences in a manner similar to naturally occurring nucleotides (e.g., peptide nucleic acids). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritiated nucleic acids, to name just two examples, are "polynucleotides" as the term is used herein. It will be appreciated that a great variety of modifications have been made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including among other things, simple and complex cells. Every nucleic acid sequence herein that encodes a polypeptide also, by reference to the genetic code, describes every possible silent variation of the nucleic acid. The term "conservatively modified variants" applies to both amino acid and nucleic acid sequences. With respect to particular nucleic acid sequences, the term "conservatively modified variants" refers to those nucleic acid sequences which encode identical or conservatively modified variants of the amino acid sequences due to the degeneracy of the genetic code. The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation.

The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" are also inclusive of modifications including, but not limited to, protein post-translational modifications (PTMs) which increase the functional diversity of the proteome by the covalent addition of functional groups or proteins, proteolytic cleavage of regulatory subunits, or degradation of entire proteins, such as phosphorylation, glycosylation, ubiquitination, nitrosylation, methylation, acetylation, lipidation and proteolysis, and other modifications of proteins well known in the art such as lipid attachment, sulfation, gamma-carboxylation of glutamic acid residues, hydroxylation and ADP-ribosylation. Within the context of the present application, oligomers (such as oligonucleotides, oligopeptides) are considered a species of the group of polymers. Oligomers have a relatively low number of monomeric units, in general 2-100, in particular 6-100.

The term "enzyme" means a protein or polypeptide that mediates biochemical action by binding to the substrate and by catalyzing the reaction that translates external cues into biological responses. So, enzymes are biological catalysts or biocatalysts that speed up biochemical reactions in living organisms. "Enzyme activity" refers to the measurement of the functional efficiency of enzymes in biological systems. The enormous catalytic activity of enzymes can perhaps best be expressed by a constant, kcat, that is variously referred to as the turnover rate, turnover frequency or turnover number. This constant represents the number of substrate molecules that can be converted to product by a single enzyme molecule per unit time (usually per minute or per second).

"Expression cassette" as used herein means a DNA sequence capable of directing expression of a particular nucleotide sequence in an appropriate host cell, such as a Rhodobacter cell, comprising a promoter operably linked to the nucleotide sequence of interest which is operably linked to termination signals. It also typically comprises sequences required for proper translation of the nucleotide sequence. The coding region usually codes for a protein of interest but may also code for a functional RNA of interest, for example antisense RNA or a non-translated RNA, in the sense or antisense direction. The expression cassette comprising the nucleotide sequence of interest may be chimeric, meaning that at least one of its components is heterologous with respect to at least one of its other components. The expression cassette may also be one which is naturally occurring but has been obtained in a recombinant form useful for heterologous expression. The expression of the nucleotide sequence in the expression cassette may be under the control of a constitutive promoter or of an inducible promoter which initiates transcription only when the host cell, such as a Rhodobacter cell, is exposed to some particular external stimulus.

The term "vector" as used herein refers to a construction comprised of genetic material designed to direct transformation of a targeted cell. A vector contains multiple genetic elements positionally and sequentially oriented, i.e., operatively linked with other necessary elements such that the nucleic acid in a nucleic acid cassette can be transcribed and when necessary, translated in the transformed cells. In particular, the vector may be selected from the group of viral vectors, (bacterio)phages, cosmids or plasmids. The vector may also be a yeast artificial chromosome (YAC), a bacterial artificial chromosome (BAC) or Agrobacterium binary vector. The vector may be in double or single stranded linear or circular form which may or may not be self transmissible or mobilizable, and which can transform Rhodobacter host organisms or cells either by integration into the cellular genome or exist extrachromosomally (e.g. autonomous replicating plasmid with an origin of replication). Included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal) cells. Preferably, the nucleic acid in the vector is under the control of, and operably linked to, an appropriate promoter or other regulatory elements for transcription in a host cell such as a microbial, e.g. bacterial, or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell. Vectors containing a nucleic acid can be prepared based on methodology known in the art per se. For instance, use can be made of a cDNA sequence encoding a polypeptide or enzyme as referred to herein operably linked to suitable regulatory elements, such as transcriptional or translational regulatory nucleic acid sequences. The term "vector" as used herein, includes reference to a vector for standard cloning work ("cloning vector") as well as to more specialized type of vectors, like an (autosomal) expression vector and a cloning vector used for integration into the chromosome of the host cell ("integration vector").

"Cloning vectors" typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector.

The term "expression vector" refers to a DNA molecule, linear or circular, that comprises a segment encoding a polypeptide or enzyme of interest under the control of (i.e. operably linked to) additional nucleic acid segments that provide for its transcription. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both. In particular an expression vector comprises a nucleotide sequence that comprises in the 5' to 3' direction and operably linked: (a) a transcription and translation initiation region that are recognized by the host organism, (b) a coding sequence for a polypeptide or enzyme of interest, and (c) a transcription and translation termination region that are recognized by the host organism. "Plasmid" refers to autonomously replicating extrachromosomal DNA which is not integrated into a microorganism's genome such as the genome of a Rhodobacter cell and is usually circular in nature.

An "integration vector" refers to a DNA molecule, linear or circular, that can be incorporated into a microorganism's genome such as the genome of a Rhodobacter cell and provides for stable inheritance of a gene encoding a polypeptide or enzyme of interest. The integration vector generally comprises one or more segments comprising a gene sequence encoding a polypeptide or enzyme of interest under the control of (i.e., operably linked to) additional nucleic acid segments that provide for its transcription. Such additional segments may include promoter and terminator sequences, and one or more segments that drive the incorporation of the gene of interest into the genome of the target cell, usually by the process of homologous recombination. Typically, the integration vector will be one which can be transferred into the target cell, but which has a replicon which is nonfunctional in that organism. Integration of the segment comprising the gene of interest may be selected if an appropriate marker is included within that segment.

One or more nucleic acid sequences encoding appropriate signal peptides that are not naturally associated with a polypeptide or enzyme to be expressed in a host cell such as a Rhodobacter cell can be incorporated into (expression) vectors. For example, a DNA sequence for a signal peptide leader can be fused in-frame to a nucleic acid sequence so that the polypeptide or enzyme is initially translated as a fusion protein comprising the signal peptide. Depending on the nature of the signal peptide, the expressed polypeptide or enzyme will be targeted differently. A secretory signal peptide that is functional in the intended host cells, for instance, enhances extracellular secretion of the expressed polypeptide or enzyme. Other signal peptides direct the expressed polypeptide or enzyme to certain organelles, like the chloroplasts, mitochondria and peroxisomes. The signal peptide can be cleaved from the polypeptide or enzyme upon transportation to the intended organelle or from the cell. It is possible to provide a fusion of an additional peptide sequence at the amino or carboxyl terminal end of the polypeptide or enzyme.

The term "functional homologue" of an amino acid sequence, or in short "homologue", as used herein, refers to a polypeptide comprising said specific sequence with the proviso that one or more amino acids are substituted, deleted, added, and/or inserted, and which polypeptide has (qualitatively) the same enzymatic functionality for substrate conversion. The term "functional homologue" of a nucleic acid sequence is used for nucleic acid sequences encoding the same polypeptide as said nucleic acid sequence.

Sequence identity, homology or similarity is defined herein as a relationship between two or more polypeptide sequences or two or more nucleic acid sequences, as determined by comparing those sequences. Usually, sequence identities or similarities are compared over the whole length of the sequences, but may, however, also be compared only for a part of the sequences aligning with each other. In the art, "identity" or "similarity" also means the degree of sequence relatedness between polypeptide sequences or nucleic acid sequences, as the case may be, as determined by the match between such sequences. Sequence identity as used herein is the value as determined by the EMBOSS Pairwise Alignment Algorithm "Needle". In particular, the NEEDLE program from the EMBOSS package can be used (version 2.8.0 or higher, EMBOSS: The European Molecular Biology Open Software Suite - Rice, P., et al. Trends in Genetics (2000) 16: 276-277; http://emboss.bioinformatics.nU) using the NOBRIEF option ('Brief identity and similarity' to NO) which calculates the "longest- identity". The identity, homology or similarity between the two aligned sequences is calculated as follows: Number of corresponding positions in the alignment showing an identical amino acid in both sequences divided by the total length of the alignment after subtraction of the total number of gaps in the alignment. For alignment of amino acid sequences the default parameters are: Matrix = Blosum62; Open Gap Penalty = 10.0; Gap Extension Penalty = 0.5. For alignment of nucleic acid sequences the default parameters are: Matrix = DNAfull; Open Gap Penalty = 10.0; Gap Extension Penalty = 0.5. Discrepancies between an enzyme, e.g., a fructokinase, a invertase, a sucrose permease, a monoterpene, or a sesquiterpene synthase as referred to herein, according to a specfic sequence or a nucleic acid according to a specific sequence on hand and a functional homologue of said enzyme or nucleic acid may in particular be the result of modifications performed, e.g. to improve a property of the enzyme or nucleic acid (e.g. improved expression) by a biological technique known to the skilled person in the art, such as e.g. molecular evolution or rational design or by using a mutagenesis technique known in the art (random mutagenesis, site-directed mutagenesis, directed evolution, gene recombination, etc.). The enzyme's or the nucleic acid's sequence may be altered, as a result of one or more natural occurring variations. Examples of such natural modifications/variations are differences in glycosylation (more broadly defined as "post-translational modifications"), differences due to alternative splicing, and single-nucleic acid polymor-phisms (SNPs). The nucleic acid sequence may be modified such that it encodes a polypeptide that differs by at least one amino acid, so that it encodes a polypeptide comprising one or more amino acid substitutions, deletions and/or insertions, which polypeptide still has the desired, e.g., (original) enzymatic activity. Further, use may be made of artificial gene -synthesis (synthetic DNA). Further, use may be made of codon optimization or codon pair optimization, e.g. based on a method as described elsewhere herein and also in WO 2008/000632 or as offered by commercial DNA synthesizing companies like DNA2.0, Geneart, and GenScript, as set forth elsewhere herein.

A host cell such as the Rhodobacter cell of the invention may be produced based on standard genetic and molecular biology techniques that are generally known in the art, e.g. as described in Sambrook, J., and Russell, D.W. "Molecular Cloning: A Laboratory Manual" 3d ed., Cold Spring Harbor Laboratory Press, Cold Spring Harbor, NY, (2001); and F.M. Ausubel et al, eds., "Current protocols in molecular biology", John Wiley and Sons, Inc., New York (1987), and later supplements thereto.

Advantageously, the host cell such as the Rhodobacter cell of the invention engineered to utilize sucrose is an organism comprising further genes for expressing the enzymes for catalyzing the reaction steps of the mevalonate pathway enabling the production of the C5 prenyl diphosphates isopentenyl diphosphate (IPP). In particular, the host cell such as the Rhodobacter cell of the invention comprises genes for expressing the following enzymes of the mevalonate pathway:
(i) an enzyme having catalytic activity in the reaction of acetoacyl-CoA with acetyl-CoA to form HMG-CoA ;
(ii) an enzyme having catalytic activity in the conversion of HMG-CoA to mevalonate;
(iii) an enzyme having catalytic activity in the phosphorylation of mevalonate to mevalonate 5-phosphate;
(iv) an enzyme having catalytic activity in the conversion of mevalonate 5-phosphate to mevalonate 5-pyrophosphate;
(v) an enzyme having catalytic activity in the conversion of mevalonate 5-pyrophosphate to IPP; and
(vi) an enzyme having catalytic activity in the reversible conversion of IPP to DMAPP.

The genes encoding enzymes (i), (ii), (iii), (iv) and (v) are usually heterologous. Preferably, one or more homologous genes encoding enzyme (vi), having catalytic activity in the reversible conversion of IPP to DMAPP, is present. In addition, one or more heterologous genes encoding an enzyme (vi) may advantageously be present.

The host cell such as the Rhodobacter cell of the invention typically comprises one or more homologous genes encoding a homologous enzyme having catalytic activity in the reaction of conversion of two molecules of acetyl-CoA to acetoacyl-CoA (hereafter 'thiolase'). The host cell only comprises a thiolase that is encoded by one or more homologous thiolase genes in case of a Rhodobacter for producing a sesquiterpene. Preferably, the host cell only comprises the thiolase encoded by one or more homologous thiolase genes in case of a Rhodobacter for producing a monoterpene. The host cell comprises a prenyl transferase having catalytic activity for the condensation of IPP and DMAPP into geranyl diphosphate (GPP). Depending on the specific prenyl transferase this enzyme also catalyzes the further condensation of IPP and GPP into farnesyl diphosphate (FPP). FPP is the substrate for sesquiterpene synthases and GPP is the substrate for monoterpene synthases. GPP and FPP synthesis can be enhanced by the expression of heterologous GPP or FPP synthases, respectively. Bacterial enzymes usually catalyze both condensations and thus are useful for the production of sesquiterpenes. Specific GPP synthases do not produce FPP and are, thus, particularly useful for the production of monoterpenes. Many GPP synthases have been described from plants and heterologous expression of such enzymes are useful for the production of monoterpenes in bacterial of fungal cells.

The terms farnesyl diphosphate and farnesyl pyrophosphate (both abbreviated as FPP) as interchangeably used herein refer to the compound 3,7, I I-trimethyl-2,6, 10- dodecatrien-I-yl pyrophosphate and include all known isomers of this compound.

The Rhodobacter cell of the invention is preferably selected from the group of Rhodobacter capsulatus and Rhodobacter sphaeroides.

The term "monoterpene synthase" is used herein for polypeptides having catalytic activity in the formation of a monoterpene from geranyl pyrophosphate, and for other moieties comprising such a polypeptide. Examples of such other moieties include complexes of said polypeptide with one or more other polypeptides, other complexes of said polypeptides (e.g. metalloprotein complexes), macromolecular compounds comprising said polypeptide and another organic moiety, said polypeptide bound to a support material, etc. The monoterpene synthase can be provided in its natural environment, i.e. within the cell in which it has been produced, or in the medium into which it has been excreted by the cell producing it. It can also be provided separate from the source that has produced the polypeptide and can be manipulated by attachment to a carrier, labeled with a labeling moiety, and the like. Suitable monoterpene synthases can be based on those known in the art. For instance, use may be made of the monoterpene synthases mentioned in, or referred to, in EP2875136, or in US 7,659,097 (of which the contents with respect to monoterpene synthases are incorporated by reference, in particular column 11, line 25 - column 15, line 5). Preferably, the enzyme having monoterpene synthase activity is selected from the group of enzymes having beta-pinene synthase activity, alpha-pinene synthase activity, myrcene synthase activity, limonene synthase activity (in particular, L-(-)limonene synthase activity), linalool synthase activity, sabinene synthase activity, bisabolene synthase activity and geraniol synthase activity. For example, SEQ ID Nos. 2, 4 and 6 of EP2875136 show the amino acid sequence of a monoterpene synthase with beta-pinene synthase activity. Specific examples of geraniol synthases are shown in SEQ ID Nos: 12, 14 and 16 of EP2875136. Specific examples of myrcene synthases are shown in SEQ ID Nos: 18 and 20 of EP2875136.

The term "sesquiterpene synthase" is used herein for polypeptides having catalytic activity in the formation of sesquiterpene from farnesyl diphosphate, and for other moieties comprising such a polypeptide. Examples of such other moieties include complexes of said polypeptide with one or more other polypeptides, other complexes of said polypeptides (e.g. metalloprotein complexes), macromolecular compounds comprising said polypeptide and another organic moiety, said polypeptide bound to a support material, etc. The sesquiterpene synthase can be provided in its natural environment, i.e. within a cell in which it has been produced, or in the medium into which it has been excreted by the cell producing it. It can also be provided separate from the source that has produced the polypeptide and can be manipulated by attachment to a carrier, labeled with a labeling moiety, and the like. Suitable sesquiterpene synthases can be based on those known in the art. For instance use may be made of the terpene synthases mentioned in or referred to, in EP2875136, or in US7,659,097 (of which the contents with respect to monoterpene synthases are incorporated by reference, in particular column 15, line 6- column 17, line 55). In particular, the sesquiterpene synthase may be selected from the group of valencene synthases, bisabolene synthases, bisabolol synthases, bergamotene synthases, farnesene synthases, zizaene synthases, zingiberene synthases, santalene synthases and patchoulol synthases. For instance, the valencene synthase may originate from Citrus × paradise. The amino acid sequences of valencene synthase are depicted in SEQ ID NO. 8, 9 or 10 of EP2875136, and a zingiberene synthase is described elsewhere herein and used in the Examples.

The enzyme such as the fructokinase, invertase, sucrose permease, monoterpene synthase, or sesquiterpene synthase referred to herein, may consist of a polypeptide referred to herein above. However, it is also possible that the enzyme such as the fructokinase, invertase, sucrose permease, monoterpene synthase, or sesquiterpene synthase referred to herein comprises at least one segment having such polypeptide, or a sequence having a high sequence identity to the amino acid sequence of said fructokinase, invertase, sucrose permease, monoterpene synthase or sesquiterpene synthase as referred to herein, such as a sequence identity of more than 70 %, of more than 75 %, of more than 80 %, of more than 85 %, of more than 90 %, or of more than 95 %, and at least one further peptide segment, such as a tag peptide.

The tag peptide is preferably selected from the group of nitrogen utilization proteins (NusA), thioredoxins (Trx) and maltose-binding proteins (MBP). Moreover small peptides with large net negative charge, as have been described by Zhang, Y-B, et al., Protein Expression and Purification (2004) 36: 207-216, can be used as tag-peptide. Particularly suitable is maltose binding protein from Escherichia coli. The tag may in particular improve productivity of the enzyme, by increasing the expression of the fructokinase, invertase, sucrose permease, and/or monoterpene synthase or sesquiterpene synthase in active form. Preferably, the fructokinase, invertase, sucrose permease, and/or monoterpene synthase or sesquiterpene synthase having a tag-peptide segment has an increased specific productivity, increased stability or an increased product specificity, in particular if the tag-peptide is selected from the group of nitrogen utilization proteins (NusA), thioredoxins (Trx) and maltose-binding proteins (MBP). For improved solubility of the tagged fructokinase, invertase, sucrose permease, and/or monoterpene synthase or sesquiterpene synthase (compared to the fructokinase, invertase, sucrose permease, monoterpene synthase or sesquiterpene synthase without the tag), the first segment of the tagged fructokinase, invertase, sucrose permease, and/or monoterpene synthase or sesquiterpene synthase is preferably bound at its C-terminus to the N-terminus of the second segment. Alternatively, the first segment of the tagged fructokinase, invertase, sucrose permease, and/or monoterpene synthase or sesquiterpene synthase is bound at its N-terminus to the C-terminus of the second segment.

The Rhodobacter cell according to the invention can be used industrially in the production of a monoterpene, preferably a monoterpene selected from the group of beta-pinene, myrcene, alpha-pinene, limonene, linalool, sabinene, bisabolene and geraniol, or in the production of a sesquiterpene, preferably a sesquiterpene selected from the group of valencene, bisabolene, bisabolol, bergamotene, farnesene, zizaene, santalene, zingiberene, and patchoulol, or in the production of a diterpene preferably sclareol, or in the production of a triterpene or tetraterpene.

In principle, the production of the monoterpene or sesquiterpene using the Rhodobacter cell of the invention can be carried out in a manner based on methodology known per se, e.g. as described in the prior art mentioned elsewhere herein.

The method of the invention for preparing a monoterpene, a sesquiterpene, a diterpene, a triterpene, a tetraterpene, a flavour, an aroma compound, an UV scavenger, a natural colorant, a natural crop protectant or an isoprenoid moiety containing compound such as coenzyme Q10, comprises culturing a Rhodobacter cell according to the invention in a culture medium comprising sucrose as carbon source, preferably sole carbon source, for the monoterpene, sesquiterpene, diterpene, triterpene, tetraterpene, flavour, aroma compound, UV scavenger, natural colorant, natural crop protectant or the isoprenoid moiety containing compound such as coenzyme Q10.

Preferably, the Rhodobacter cell according to the invention may be used in a fermentative production of the montoterpene or sesquiterpene or diterpene or triterpene or tetraterpene, or it may be used to produce a monoterpene synthase or sesquiterpene synthase or diterpene synthase or triterpene synthase or tetraterpene synthase, which can thereafter then be used for synthesis of the desired terpenoid.

Advantageously, the monoterpene or sesquiterpene or diterpene or triterpene or tetraterpene is produced in a fermentative process, i.e. in a method comprising cultivating the Rhodobacter host cells of the invention in a culture medium under conditions wherein the monoterpene synthase or sesquiterpene synthase or diterpene synthase or triterpene synthase or tetraterpene synthase is expressed. The actual reaction catalyzed by the monoterpene synthase or sesquiterpene synthase or diterpene synthase or triterpene synthase or tetraterpene synthase typically takes place intracellularly.

It should be noted that the term "fermentative" is used herein in a broad sense for processes wherein use is made of a culture of an organism to synthesize a compound from a suitable feedstock (e.g. a carbohydrate such as sucrose, an amino acid source, a fatty acid source). Thus, fermentative processes as meant herein are not limited to anaerobic conditions, and extended to processes under aerobic conditions. Suitable feedstocks are generally known for Rhodobacter host cells. Suitable conditions may be based on known methodology for Rhodobacter host cells, e.g. described in WO 2011/074954 (in particular page 68 (examples, general part, shake-flask procedure) or in EP 2875136, the information disclosed herein, common general knowledge and optionally some routine experimentation.

In principle, the pH of the reaction medium (culture medium) used in a method according to the invention may be chosen within wide limits, as long as the Rhodobacter host cell is active and displays a wanted specificity under the pH conditions. To give a specific example, in case the method includes the use of Rhodobacter cells, for expressing the valencene synthase, the pH is selected such that the cells are capable of performing their intended function or functions. The pH may in particular be chosen within the range of four pH units below neutral pH and two pH units above neutral pH, i.e. between pH 3 and pH 9 in case of an essentially aqueous system at 25°C. Good results have, e.g., been achieved in an aqueous reaction medium having a pH in the range of 6.8 to 7.5.

A system is considered aqueous if water is the only solvent or the predominant solvent (> 50 wt. %, in particular > 90 wt. %, based on total liquids), wherein e.g. a minor amount of alcohol or another solvent (< 50 wt. %, in particular < 10 wt. %, based on total liquids) may be dissolved (e.g. as a carbon source, in case of a full fermentative approach) in such a concentration that microorganisms which are present remain active.

The reaction conditions can be aerobic, oxygen-limited or anaerobic. Anaerobic conditions are herein defined as conditions without any oxygen or in which substantially no oxygen is consumed by the cultured cells, in particular a microorganism, and usually corresponds to an oxygen consumption of less than 5 mmol/l.h, preferably to an oxygen consumption of less than 2.5 mmol/l.h, or more preferably less than 1 mmol/l.h. Aerobic conditions are conditions in which a sufficient level of oxygen for unrestricted growth is dissolved in the medium, able to support a rate of oxygen consumption of at least 10 mmol/l.h, more preferably more than 20 mmol/l.h, even more preferably more than 50 mmol/l.h, and most preferably more than 100 mmol/l.h. Oxygen-limited conditions are defined as conditions in which the oxygen consumption is limited by the oxygen transfer from the gas to the liquid. The lower limit for oxygen-limited conditions is determined by the upper limit for anaerobic conditions, i.e. usually at least 1 mmol/l.h, and in particular at least 2.5 mmol/l.h, or at least 5 mmol/l.h. The upper limit for oxygen-limited conditions is determined by the lower limit for aerobic conditions, i.e. less than 100 mmol/l.h, less than 50 mmol/l.h, less than 20 mmol/l.h, or less than to 10 mmol/l.h.

Whether conditions are aerobic, anaerobic or oxygen-limited is dependent on the conditions under which the method is carried out, in particular by the amount and composition of ingoing gas flow, the actual mixing/mass transfer properties of the equipment used, the type of Rhodobacter used and the microorganism density. In principle, the temperature used is not critical, as long as the cells, show substantial activity. Generally, the temperature may be at least 0°C, in particular at least 15°C, more in particular at least 20°C. A desired maximum temperature depends upon the enzymes and the cells. The temperature is generally 70°C or less, preferably 50°C or less, more preferably 40°C or less, in particular 35°C or less.

In particular, if the catalytic reaction whereby monoterpene or sesquiterpene or diterpene or triterpene or tetraterpene is formed, is carried out outside a host cell, a reaction medium comprising an organic solvent may be used in a high concentration {e.g. more than 50 %, or more than 90 wt. %, based on total liquids), in case the monoterpene or sesquiterpene or diterpene or triterpene or tetraterpene synthase that is used retains sufficient activity and specificity in such a medium.

If desired, the monoterpene or sesquiterpene or diterpene or triterpene or tetraterpene produced in a method according to the invention, or a further compound into which the monoterpene or sesquiterpene or diterpene or triterpene or tetraterpene has been converted after its preparation (such as nootkatone prepared from valencene), is recovered from the reaction medium, wherein it has been made. A suitable method usually is liquid-liquid extraction with an extracting liquid that is non-miscible with the reaction medium.

In an advantageous embodiment, the monoterpene or sesquiterpene or diterpene or triterpene or tetraterpene (or a further product) is produced in a reactor comprising a first liquid phase (the reaction phase), said first liquid phase containing Rhodobacter cells according to the invention in which cells the monoterpene or sesquiterpene or diterpene or triterpene or tetraterpene (or a further product) is produced, and a second liquid phase (organic phase that remains essentially phase-separated with the first phase when contacted), said second liquid phase being the extracting phase, for which the formed product has a higher affinity. This method is advantageous in that it allows in situ product recovery. Also, it contributes to preventing or at least reducing potential toxic effects of the monoterpene or sesquiterpene or diterpene or triterpene or tetraterpene (or a further product) to the cells, because due to the presence of the second phase, the monoterpene or sesquiterpene or diterpene or triterpene or tetraterpene (or a further product) concentration in the reaction phase may be kept relatively low throughout the process. Finally, there are strong indications that the extracting phase contributes to extracting the monoterpene or sesquiterpene or diterpene or triterpene or tetraterpene (or further product) out of the reaction phase.

In a preferred method of the invention, the extracting phase forms a layer on top of the reaction phase or is mixed with the reaction phase to form a dispersion of the reaction phase in the extracting phase or a dispersion of the extracting phase in the reaction phase. Thus, the extracting phase not only extracts product from the reaction phase, but also helps to reduce or completely avoid losses of the formed product from the reactor through the off-gas, that may occur if the monoterpene or sesquiterpene or diterpene or triterpene or tetraterpene is produced in the (aqueous) reaction phase or excreted into the (aqueous) reaction phase. Generally, monoterpenes and sesquiterpenes or diterpenes or triterpenes or tetraterpenes are poorly soluble in water and, therefore, easily volatilize from water. It is contemplated by the invention that a monoterpene or sesquiterpene or diterpene or triterpene or tetraterpene dissolved in the organic phase is at least substantially prevented from volatilization.

Suitable liquids for use as extracting phase combine a lower density than the reaction phase with a good biocompatibility (no interference with the viability of living cells), low volatility, and near absolute immiscibility with the aqueous reaction phase. Examples of suitable liquids for this application are liquid alkanes like decane, dodecane, isododecane, tetradecane, and hexadecane or long-chain aliphatic alcohols like oleyl alcohol, and palmitoleyl alcohol, or esters of long-chain fatty acids like isopropyl myristate, and ethyl oleate (see e.g. Asadollahi et al. (Biotechnol. Bioeng. (2008) 99: 666-677), Newman et al. (Biotechnol. Bioeng. (2006) 95: 684-691) and WO 2009/042070).

The monoterpene or sesquiterpene or diterpene or triterpene or tetraterpene produced in accordance with the method of the invention may be used as/for producing of a flavour or fragrance, aroma compound, UV scavenger, natural colorant, natural crop protectant, as an insect repellent, or coenzyme Q10, and may also be be used as a starting material for another compound, in particular another flavour or fragrance or aroma compound. To give an example, valencene may be converted into nootkatone. The conversion of valencene into nootkatone may be carried out intracellularly, or extracellularly. If this preparation is carried out inside a cell, the nootkatone is isolated from the host cell after its production. Suitable manners of converting valencene to nootkatone are known in the art, e.g. as described in Fraatz et al. Appl.Microbiol.Biotechnol (2009) 83: 35-41, of which the contents are incorporated by reference, or the references cited therein.

### FIGURES

The Figures show:
**Figure 1****:** Plasmids pBBR-IFT, pBBR-IF and pBBR-IT, pBBR-TF.
**Figure 2****:** Plasmids pRK415-IFT, pBBR-ZS.
**Figure 3****:** GC-MS chromatogram of zingiberene production with strain Cs_pRK415-IFT/pBBR-ZS.
**Figure 4****:** Terpene production of Cs_pRK415-IFT/pBBR-ZS strain utilizing sucrose compared to the Cs_pRK415-EV/pBBR-ZS and Cs_pBBR-ZS strains utilizing glucose as carbon source.
**Figure 5****:** OD600 measurement Cs_pRK415-IFT/pBBR-ZS strain utilizing glucose and sucrose.
**Figure 6****:** Sugar consumption of Cs_pRK415-IFT/pBBR-ZS strain utilizing glucose and sucrose.
**Figure 7****:** Terpene production of Cs_pRK415-IFT/pBBR-ZS strain utilizing glucose and sucrose.

### SEQUENCES

The following sequences are referred to throughout the specification and in the accompanying sequence protocol:

| | |
|---|---|
| SEQ ID NO: 1: | codon optimized nucleic acid sequence encoding the invertase cscA from E. coli KO11 (nucleotide Inv_CO) |
| SEQ ID NO: 2: | amino acid sequence of the invertase cscA from E. coli KO11 (protein Inv_CO) |
| SEQ ID NO: 3: | codon optimized nucleic acid sequence encoding the fructokinase cscK from E. coli KO11 (nucleotide Frk_CO) |
| SEQ ID NO: 4: | amino acid sequence of the fructokinase cscK from E. coli KO11 (protein Frk_CO) |
| SEQ ID NO: 5: | codon optimized nucleic acid sequence encoding the sucrose permease cscB from E. coli KO11 (nucleotide Trans_CO) |
| SEQ ID NO: 6: | amino acid sequence of the sucrose permease cscB from E. coli KO11 (protein Trans_CO) |
| SEQ ID NO: 7: | nucleic acid sequence of the Pppa promoter |
| SEQ ID NO: 8: | RBS sequence |
| SEQ ID NO: 9: | nucleic acid sequence of the Prplm promoter |
| SEQ ID NO: 10: | codon optimized nucleic acid sequence encoding the zingiberene synthase from nootka cypress (Callitropsis nootkatensis) (nucleotide zingiberene synthase) |
| SEQ ID NO: 11: | amino acid sequence of the zingiberene synthase from nootka cypress (Callitropsis nootkatensis) (protein zingiberene synthase) |

### EXAMPLES

The Examples shall merely illustrate the invention. They shall not, whatsoever, be construed as limiting the scope.

### Example 1: Construction of sucrose utilization pathway plasmids pBBR-IFT, pBBR-IT, pBBR-IF and pBBR-TF

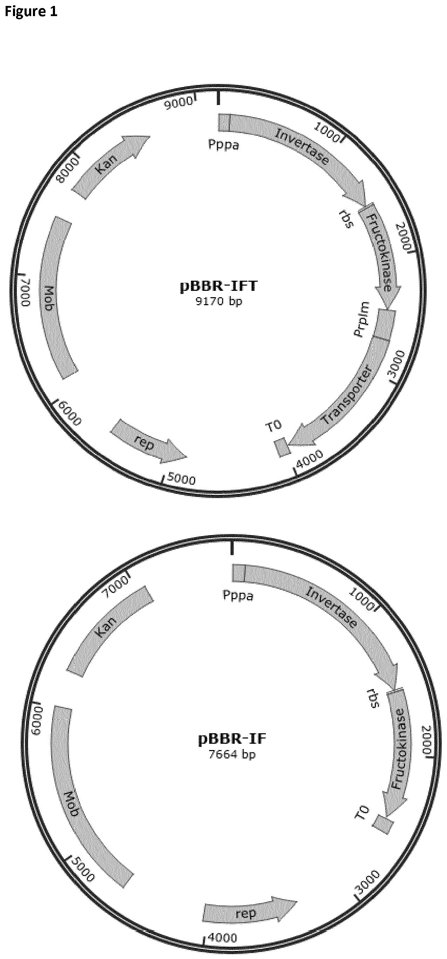

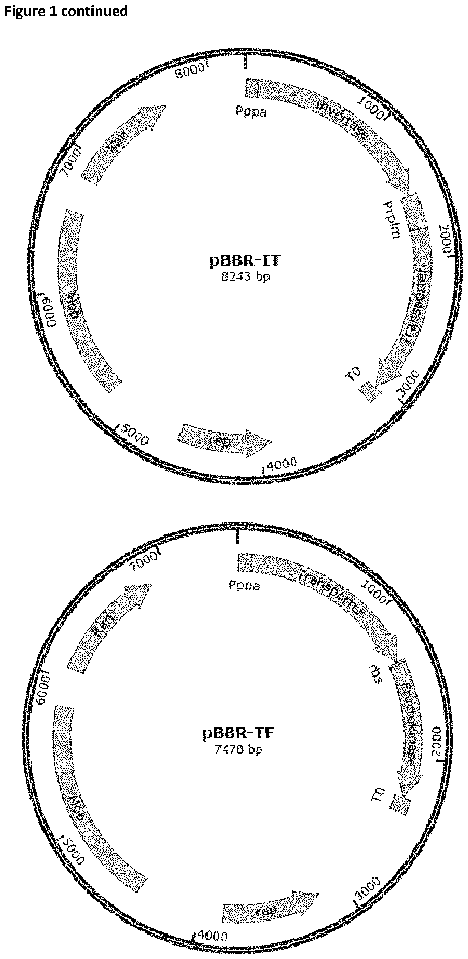

For the expression of the sucrose utilization genes from E. coli KO11 in C. sphaeroides, the full length ORFs of the invertase cscA, fructokinase cscK and sucrose permease cscB were optimized in terms of codon usage for C. sphaeroides and custom synthesized by Genscript Europe (Rijswijk, The Netherlands). SEQ ID No. 1 shows the codon optimized nucleic acid sequence encoding the invertase cscA from E. coli KO11 (nucleotide Inv_CO). SEQ ID No. 2 shows the amino acid sequence of the invertase cscA from E. coli KO11 (protein Inv_CO). SEQ ID No. 3 shows the codon optimized nucleic acid sequence encoding the fructokinase cscK from E. coli KO11 (nucleotide Frk_CO). SEQ ID No. 4 shows the amino acid sequence of the fructokinase cscK from E. coli KO11 (protein Frk_CO). SEQ ID No. 5 shows the codon optimized nucleic acid sequence encoding the sucrose permease cscB from E. coli KO11 (nucleotide Trans_CO). SEQ ID No. 6 shows the amino acid sequence of the sucrose permease cscB from E. coli KO11 (protein Trans_CO). The constructs were delivered cloned into plasmid pUC57. The sequence of the promoter Pppa (SEQ ID No. 7) was added at the 5'-end of the invertase gene to obtain plasmid pUC57_Pppa-lnv_CO. A synthetic ribosomal binding site (SEQ ID No. 8) was added at the 5'-end of the fructokinase gene to obtain plasmid pUC57_rbsFrk_CO. Sucrose permease gene and promoter Prplm (SEQ ID No. 9) were ordered separately to obtain plasmids pUC57_Trans_CO and pUC57_Prplm. Golden gate compatible restriction sites were added as flanks in all cases to allow consequent operon assembly. Synthetic parts were then assembled into a pBBR-based expression vector containing the kanamycine resistance marker. Synthetic operons were constructed using Golden Gate assembly using Type IIS enzyme Bsal. To this end 37.5 ng of donor plasmids pUC57_Pppa-Inv_CO, pUC57_rbsFrk_CO, pUC57_Prplm, pUC57_Trans_CO and the acceptor plasmid pBBR were added to a 1.5 ml microcentrifuge tube containing the Golden Gate assembly mix (New England Biolabs). The assembly reactions were incubated for 40 cycles for 1 min at 37 °C and 1 min at 16 °C, followed by an incubation for 5 min at 60 °C and 10 min at 80 °C and a final hold at 4 °C. The Golden Gate assembly mixture was transformed into E. coli DH5α cells. By this procedure, the invertase and fructokinase genes were assembled into a synthetic operon under the control of the Pppa promoter and were interspaced by a synthetic ribosomal binding site. The sucrose permease gene was assembled into a separate operon under the control of the Prplm promoter. The resulting plasmid pBBR-IFT (FIG. 1) was next transformed to the E. coli S17-1 strain. Strain ATCC35053 was purchased from the American Type Culture Collection (ATCC-Manassas, Va., USA-www.atcc.org); number 35053; Rhodobacter sphaeroides (van Niel) Imhoff et al., isolated from a sewage settling pond in Indiana and deposited as Rhodopseudomonas sphaeroides van Niel. Transfer of pBBR-IFT from S17-1 to C. sphaeroides ATCC35053 by conjugation was performed using standard procedures. Empty vector pBBR-EV was conjugated to C. sphaeroides ATCC35053 as a control. By this procedure strain Cs_pBBR-IFT and Cs_pBBR-EV were generated.

To study the importance of the three sucrose utilization genes for sucrose assimilation in C. sphaeroides, next plasmids were constructed using Golden Gate assembly using the procedure above in which one of the three pathway genes was omitted. The Golden Gate assembly mixture was transformed into E. coli DH5α cells. Plasmid pBBR-IT (FIG. 1) was assembled to contain the codon optimized invertase gene, under the control of the Pppa promoter (SEQ ID No. 7) and the codon optimized sucrose permease gene under the control of the Prplm promoter (SEQ ID No. 9). Plasmid pBBR-IF (FIG. 1) was assembled to contain the codon optimized invertase gene and codon optimized fructokinase gene, both under the control of the Pppa promoter (SEQ ID No. 7) and interspaced with the synthetic ribosomal binding site (SEQ ID No. 8). Plasmid pBBR-TF (FIG. 1) was assembled to contain the codon optimized sucrose permease gene and the codon optimized fructokinase gene both under the control of the Pppa promoter (SEQ ID No. 7) and interspaced with the synthetic ribosomal binding site (SEQ ID No. 8). Plasmids pBBR-TF, pBBR-IT and pBBR-IF were next transformed into the E. coli S17-1. Transfer of the plasmids from E. coli S17-1 to C. sphaeroides ATCC35053 by conjugation was performed using standard procedures. By this procedure strains Cs_pBBR-TF, Cs_pBBR-IT and Cs_pBBR-IF were generated.

### Example 2: Determination of C. sphaeroides growth rates utilizing glucose and sucrose

To determine the growth rates of C. sphaeroides ATCC35053 strains utilizing different carbon sources, the strains were inoculated from glycerol stock into 10 mL of LB medium and incubated in a shaking incubator overnight at 250 rpm at 30 °C. Seed cultures were inoculated at the OD600 of 0.005 in 50 mL of modified Sistrom's minimal medium (SMM medium). The medium contained 10 g/L of carbon source and 3.5 g/L of NH4Cl as nitrogen source. Moreover, the SMM contained (per liter): 3.48 g KH2PO4, 0.1 g glutamic acid, 0.04 g I-aspartic acid, 0.5 g NaCl, 0.02 g nitrilotriacetic acid, 0.3 g MgSO4 7H2O, 0.0334 g CaCl2·2H2O, 0.002 g FeSO4·7H2O, and 0.0002 g (NH4)6Mo7O24. pH was set at pH 7. Trace elements were added (0.01% v/v) from a stock solution containing: 17.65 g/L disodium EDTA, 109.5 g/L ZnSO4·7H2O, 50 g/L FeSO4·7H2O, 15.4 g/L MnSO4·7H2O, 3.92 g/L CuSO4·5H2O, 2.48 g/L Co(NO3)2·6H2O, and 0.0114 g/L H3BO3. Vitamins were added (0.01% v/v) from a stock containing: 10 g/L nicotinic acid, 5 g/L thiamine HCl, and 0.1 g/L biotin. Cultivations were performed in 50mL culture broth in 250 mL Erlenmeyer flasks in duplicates. The flasks were incubated for 40-43 h at 30° C in a shaking incubator at 250 rpm. OD600 measurements were carried out in regular intervals during the exponential phase for the calculation of the specific growth rate. Firstly, the native growth rates of C. sphaeroides ATCC35053 strain utilizing glucose and sucrose as carbon source were determined (Table 1).

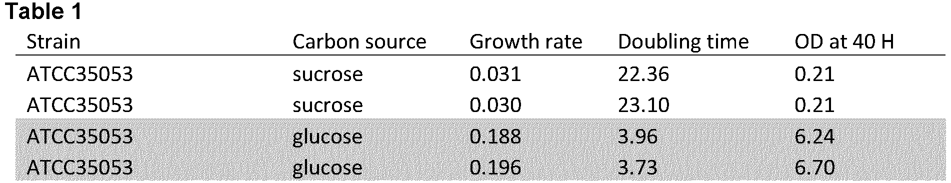

The measurements show that the strain shows very poor growth when sucrose is given as a carbon source, with observed growth rates of 0.03 h-1, compared to 0.19 h-1 for glucose as carbon source.

To determine the growth rate of the C. sphaeroides ATCC35053 strain containing the sucrose utilization genes, C. sphaeroides strains Cs_pBBR-IFT and Cs_pBBR-EV were inoculated from corresponding glycerol stocks into 10 mL of LB medium supplemented with 50 mg/L kanamycine and incubated in a shaking incubator overnight at 250 rpm at 30 °C. Seed cultures were inoculated at the OD600 of 0.005 in 50 mL of modified Sistrom's minimal medium. When growing the strain Cs_pBBR-EV a growth rate of 0.16 h-1 was observed in SMM medium containing glucose (Table 2).

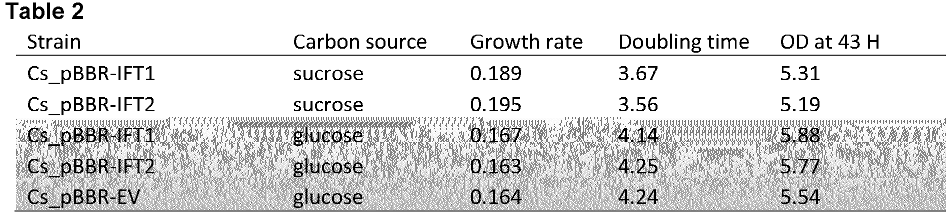

When growing the strain Cs_pBBR-IFT in SMM medium containing glucose a growth rate of 0.16 was observed, which was comparable to the control strain harboring the empty vector. When sucrose was provided as carbon source, surprisingly, the strain Cs_pBBR-IFT showed enhanced growth rate at 0.19 h-1 compared to growth with glucose as carbon source (Table 2).

In the following experiment growth rates of strains Cs_pBBR-IT, Cs_pBBR-IF and Cs_pBBR-TF were compared to strain Cs_pBBR-IFT. Strains were inoculated from corresponding glycerol stocks into 10 mL of LB medium supplemented with 50 mg/L kanamycine and incubated in a shaking incubator overnight at 250 rpm at 30 °C. Seed cultures were inoculated at the OD600 of 0.005 in 50 mL of modified Sistrom's minimal medium. The growth rates in this experiment were slightly lower than measured in previous experiments. Growth of all four strains was comparable when utilizing glucose as a carbon source and was ranging between 0.12-0.13 h-1. When utilizing sucrose as a carbon source large differences between strains were observed, showing growth rates of 0.16 h-1, 0.17 h-1 ,0.08 h-1 and 0.04 h-1 for strains Cs_pBBR-IFT, Cs_pBBR-IT, Cs_pBBR-IF and Cs_pBBR-TF, respectively (Table 3).

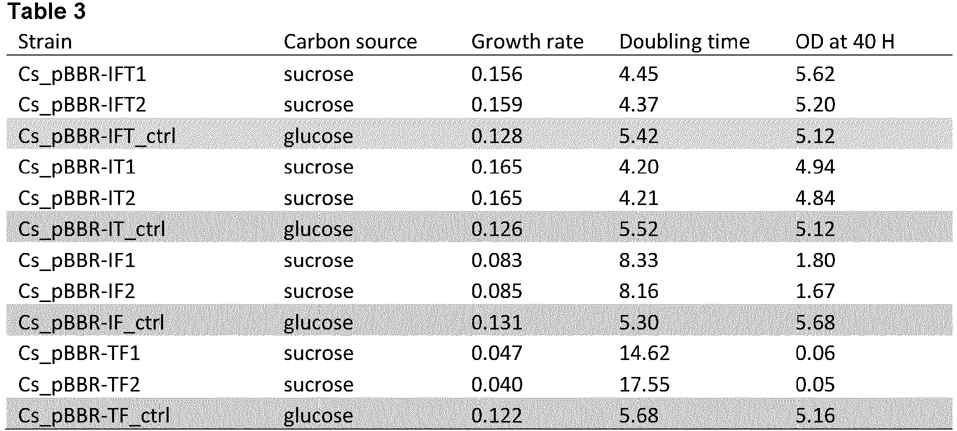

This was demonstrating that the expression of the fructokinase cscK was not necessary for sucrose utilization by C. sphaeroides in the SMM minimal medium. However, the omission of the sucrose permease cscB in strain Cs_BBR-IF and invertase cscA in strain Cs_pBBR-TF led to severely hampered growth of the strains when utilizing sucrose as a sole carbon source.

### Example 3: Construction of sucrose utilization plasmid pRK415-IFT and zingiberene production plasmid pBBR-ZS

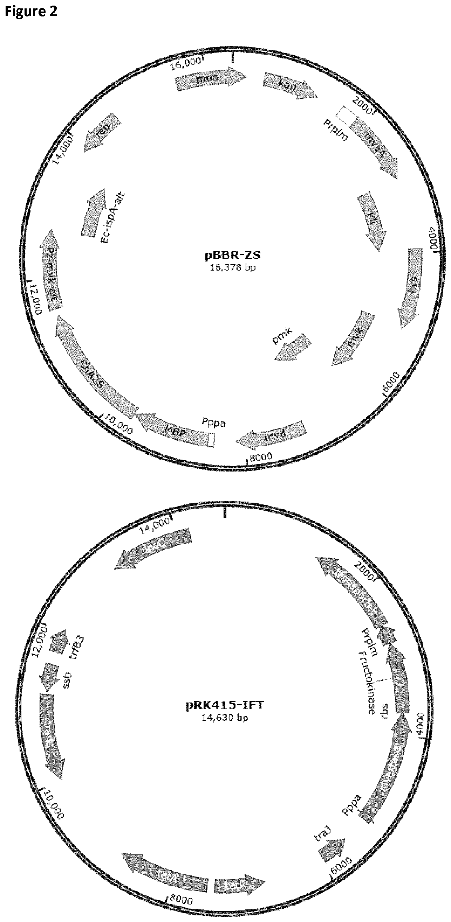

To test the capacity of C. sphaeroides to produce terpenes while using sucrose as a carbon source, two new constructs were built. To allow selection of the invertase, fructokinase and sucrose permease encoding operons, by tetracyclin instead of kanamycin, the IFT synthetic construct was excised from the plasmid pBBR-IFT using Hindlll and BamHl restriction enzymes and ligated into the multiple cloning site of vector pRK415, containing a tetracycline resistance marker, to obtain the plasmid pRK415-IFT (FIG. 2). Next, the CnZS gene encoding the zingiberene synthase from the nootka cypress (Callitropsis nootkatensis) was custom synthesized, and codon optimized for expression in C. sphaeroides by Genscript USA Inc. SEQ ID No. 10 shows the codon optimized nucleic acid sequence encoding the zingiberene synthase from nootka cypress (Callitropsis nootkatensis) (nucleotide zingiberene synthase). SEQ ID No. 11 shows the amino acid sequence of the zingiberene synthase from nootka cypress (Callitropsis nootkatensis) (protein zingiberene synthase). The CnZS sequence was cloned in plasmid SPppa-MBP with the kanamycin resistance marker, as described in WO 2019/045568 A1 (Isobionics B.V.), and a mevalonate pathway was included, as described in WO 2019/045568 A1 as well, using BamHl and Hindlll restriction sites. The resulting ligation mixture was transformed into E. coli S17-1 cells. By this procedure, plasmid pBBR-ZS was obtained (FIG. 2). Transfer of pBBR-ZS, from E. coli S17-1 to C. sphaeroides strain ATCC35053 by conjugation was performed using standard procedures (cf. US 9,260,709 B2, Isobionics B.V.) Next, plasmid pRK415-IFT and empty pRK415-EV plasmid were transferred to the C. sphaeroides ATCC35053 strain already containing the pBBR-ZS plasmid by conjugation. By this procedure strains Cs_pBBR-ZS and Cs_pRK415-IFT/pBBR-ZS and Cs_ pRK415-EV/pBBR-ZS were generated.

### Example 4: Production of terpenes utilizing sucrose as a carbon source

Seed cultures were performed in 100 ml shake flasks without baffles with 20 ml RS102 medium containing 30 g/L glucose and supplemented with 50 mg/L kanamycin when culturing strains harboring the pBBR-ZS plasmid and 50 mg/L kanamycin and 1.5 mg/L tetracycline for strains containing the combination of pRK415-IFT/pBBR-ZS and pRK415-EV/pBBR-ZS plasmids. A loop of glycerol stock was added to the medium and seed culture flasks were grown for 48 hours at 30°C in a shaking incubator at 250 rpm. At the end of the incubation OD600 of the culture was measured in order to calculate the exact volume of culture to be transferred to the main culture to obtain a starting OD600 value of 0.05 in 20 ml medium for all strains. Shake flask experiments were performed in 100 ml shake flasks without baffles with 20 ml RS102 medium containing either 30 g/L glucose or 28.5 g/L sucrose, supplemented with appropriate antibiotics and overlaid with 2 mL sterile n-dodecane. The flasks were kept for 88 hours at 30°C in a shaking incubator at 250 rpm. Shake flask experiments were performed in duplicates.

RS102 medium was prepared as described in WO 2019/045568: 20 g/L Yeast extract, 0.5 g/L MgSO4 7H2O and 0.5 g/L NaCl were dissolved in distilled water, pH was adjusted to 7.4 with NaOH, distilled water is added to a volume of 930 ml, and the medium was sterilised by autoclaving. Two ml of sterile filtered microelements (80 g/L (NH4)2Fe(SO4)2 6H2O; 6 g/L ZnSO4-7H2O; 2 g/L MnSO4 H2O; 0.2 g/L NiSO4-6H2O; 2 g/L vitamin C), and 2 ml of sterile filtered CaFe solution (75 g/L CaCl2·2H2O; 5 g/L FeCl3·6H2O; 3.75 ml HCl (37%) were added to the sterilized medium. The medium was supplemented with either 30 g/L glucose or 28.5 g/L sucrose.

Cultures were collected after 88h of incubation in 50 mL PP tubes. Dodecane was separated from the cultures by centrifugation for 15 min 3600 rpm. The n-dodecane layer was then transferred to a microcentrifuge tube and saved for a later GC-MS analysis. Next, 12 uL of dodecane was added to a glass 4 mL vial and 4 mL of ethyl acetate were added to the vial resulting in a 333 × dilution of dodecane. The extracts were dried over a Na2SO4 column prepared in a glass wool plugged glass pipette. Gas chromatography was performed on gas chromatograph (5890 series II; Hewlett-Packard, Palo Alto, CA, USA) equipped with a 30 m × 0.25 mm, 0.25 mm film thickness column (ZB-5; Phenomenex) using helium as carrier gas at flow rate of 1 mL/min. The injector was used in splitless mode with the inlet temperature set to 250 °C. The initial oven temperature of 45 °C was increased after 1 min to 300 °C at a rate of 10 °C/min and held for 5 min at 300 °C. The GC was coupled to a mass-selective detector (model 5972A; Hewlett-Packard), scanning from 45 to 350 atomic mass units.

Fermentation substrate consumption was measured by HPLC. A Waters HPLC system (Waters model e2695) was used equipped with a refractive index detector (Waters model 2414) and a Shodex KC-811 300 × 8 mm column, at 60°C with 3mM H2SO4 as mobile phase and a flow rate of 1 ml/min followed by detection using a Waters 2414 differential refractometer (Waters, USA). Prior to analysis, the supernatant was diluted 1:1 with 1 M H2SO4, centrifuged, and filtered (pore size of 0.2 µm) to remove solids and precipitated proteins. As internal standard, 100 mM valeric acid in 1 M H2SO4 was used.

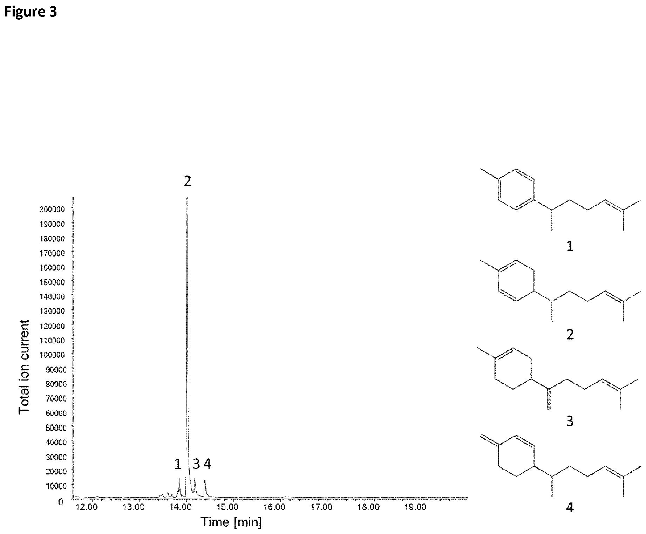

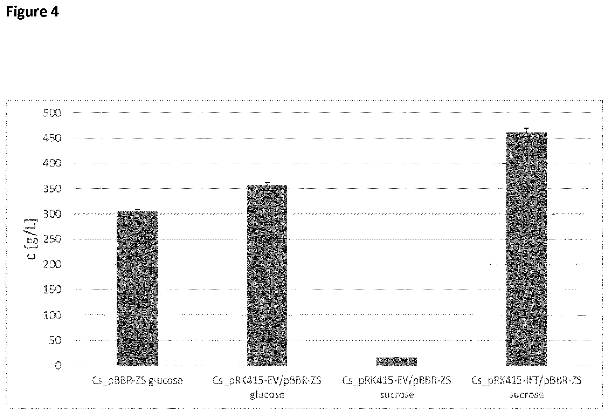

The GC-MS analysis revealed that zingiberene is produced as the main product (peak 2) and alpha-curcumene, (peak 1), beta-bisabolene (peak 3) and beta-sesquiphellandrene (peak 4) are seen as side products upon expression of the CnZS (FIG. 3), as identified by comparison with the NIST and in house spectral libraries. Surprisingly, strain Cs_pRK415-IFT/pBBR-ZS utilizing sucrose produced significantly higher yield of zingiberene, resulting in 29% and 51% titer increase compared to strains Cs_pRK415-EV/pBBR-ZS and Cs_pBBR-ZS utilizing glucose (FIG. 4). As expected for strain Cs_pRK415-EV/pBBR-ZS which was not able to utilize sucrose, only a minor zingiberene production was observed upon cultivation in RS102 medium supplemented with sucrose. Sugar consumption measurements showed that all sugar was consumed at 88 h when strain Cs_pRK415-IFT/pBBR-ZS was cultivated with sucrose and when strains Cs_pRK415-EV/pBBR-ZS and Cs_pBBR-ZS were cultivated with glucose as carbon source. Sucrose concentration of app. 30 g/L was measured after 88h in Rs102 medium supplemented with sucrose for strain Cs_pRK415-EV/pBBR-ZS, which was equal to the concentration added to the medium at the startpoint, indicating that sucrose was indeed not utilized by this strain.

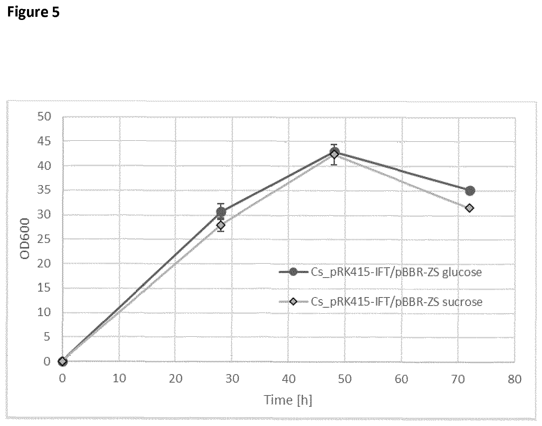

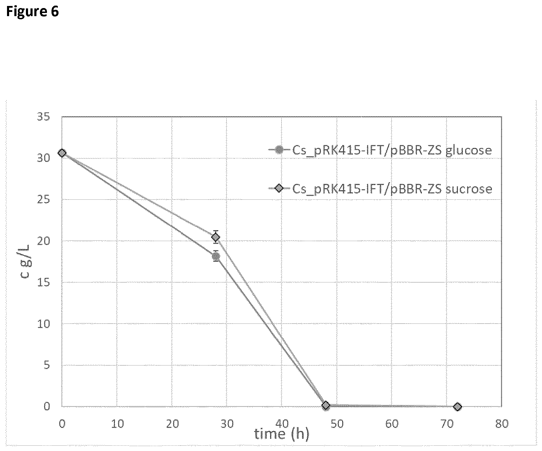

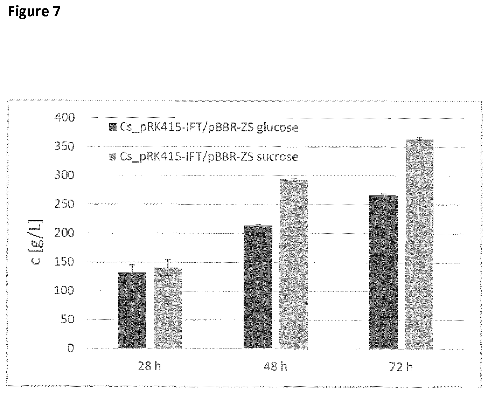

In a follow-up shake flask cultivation strain Cs_pRK415-IFT/pBBR-ZS was grown in RS 102 medium as described above and the growth was terminated at either 28h, 48h or 72h. Shake flask was performed in duplicates. The strain growth, sugar consumption and terpene production were evaluated when utilizing glucose or sucrose as a carbon source. OD600 measurements reveal that the maximal OD600 was observed at the 48h measurement for both carbon sources (FIG. 5). Sugar consumption measurement shows that all sugar was consumed at 48h in both culture broth containing glucose and sucrose (FIG. 6). When measuring the terpene production equal terpene production is observed at 28h when utilising glucose or sucrose. Surprisingly, a significantly higher terpene production is observed at 48 h and at 72 h when utilizing sucrose compared to glucose, leading to a 37% increase in terpene titer both at the 48 h and 72 h time point (FIG. 7).

In another experiment, thick juice (600 g/kg sucrose) or molasse (450 g/kg sucrose) from sugarbeet processing were used in the culture medium, at similar carbohydrate molar concentrations as pure sucrose (20 ml RS102 medium containing 28.5 g/L sucrose). In both cases, increases of more than 20% in terpene titer were observed at 72h hours of cultivation, compared to glucose. This experiment demonstrates that also less refined forms of sucrose can be used in the current invention to promote terpene formation.

## Claims

1. A Rhodobacter cell engineered to utilize sucrose, comprising:
(i) a heterologous enzyme having invertase activity;
(ii) a heterologous enzyme having sucrose permease activity; and, optionally,
(iii) a heterologous enzyme having fructokinase activity.

2. The Rhodobacter cell of claim 1, comprising:
(i) a heterologous enzyme having invertase activity which comprises or consists of an amino acid sequence as shown in SEQ ID NO. 2, or an amino acid sequence having at least 70% sequence identity to SEQ ID No. 2;
(ii) a heterologous enzyme having sucrose permease activity which comprises or consists of an amino acid sequence as shown in SEQ ID NO. 6, or an amino acid sequence having at least 70% sequence identity to SEQ ID No. 6; and, optionally,
(iii) a heterologous enzyme having fructokinase activity which comprises or consists of an amino acid sequence as shown in SEQ ID NO. 4, or an amino acid sequence having at least 70% sequence identity to SEQ ID No. 4.

3. The Rhodobacter cell of claim 1 or 2, comprising:
(i) a heterologous nucleic acid sequence as shown in SEQ ID No. 1, or a heterologous nucleic acid sequence having at least 70% sequence identity to SEQ ID No. 1, wherein said heterologous nucleic acid sequence encodes an enzyme having invertase activity of SEQ ID No. 2;
(ii) a heterologous nucleic acid sequence as shown in SEQ ID No. 5, or a heterologous nucleic acid sequence having at least 70% sequence identity to SEQ ID No. 5, wherein said heterologous nucleic acid sequence encodes an enzyme having sucrose permease activity of SEQ ID No. 6; and, optionally,
(iii) a heterologous nucleic acid sequence as shown in SEQ ID No. 3, or a heterologous nucleic acid sequence having at least 70% sequence identity to SEQ ID No. 3, wherein said heterologous nucleic acid sequence encodes an enzyme having fructokinase activity of SEQ ID No. 4.

4. The Rhodobacter cell of claim 3, wherein the heterologous nucleic acid sequence as defined in claim 3(i), 3(ii) and 3(iii) is codon-optimized for expression in Rhodobacter.

5. The Rhodobacter cell of claim 3 or 4, wherein the heterologous nucleic acid sequence as defined in claim 3(i), 3(ii) and 3(iii) is under the control of a constitutive promoter.

6. The Rhodobacter cell of claim 5, wherein the constitutive promoter driving the expression of the heterologous nucleic acid sequence encoding the enzyme having invertase activity and the optional heterologous nucleic acid sequence encoding the enzyme having fructokinase activity is the Pppa promoter, preferably comprising a sequence as shown in SEQ ID No. 7, or the PcrtE promoter, and the constitutive promoter driving the expression of the heterologous nucleic acid sequence encoding the enzyme having sucrose permease activity is the Prplm promoter, preferably comprising a sequence as shown in SEQ ID No. 9.

7. The Rhodobacter cell of any one of claims 1 to 6, wherein the Rhodobacter cell exhibits (i) an increased growth rate on sucrose as carbon source, compared to the growth rate on glucose as carbon source, and/or (ii) improved sucrose utilization compared to a wildtype Rhodobacter cell.

8. The Rhodobacter cell of claim 7, wherein sucrose is the sole carbon source and sucrose is refined sucrose, preferably from sugarcane or sugarbeet, less-refined sucrose sugar streams, molasses, thick juice or sugarcane juice.

9. The Rhodobacter cell of any one of claims 1 to 8, further comprising:
(i) nucleic acid sequences encoding enzymes of a mevalonate pathway for making isoprenyl pyrophosphate (IPP) and its isomer dimethylallyl pyrophosphate (DMAPP),
(ii) a nucleic acid sequence encoding an enzyme having catalytic activity for the condensation of IPP and DMAPP into geranyl diphosphate (GPP), and
(iii) a nucleic acid sequence encoding an enzyme having monoterpene synthase activity in the conversion of GPP into a monoterpene.

10. The Rhodobacter cell of claim 9, wherein the enzyme having monoterpene synthase activity is selected from the group of enzymes having beta-pinene synthase activity, alpha-pinene synthase activity, myrcene synthase activity, limonene synthase activity, linalool synthase activity, sabinene synthase activity, bisabolene synthase activity, and geraniol synthase activity.

11. The Rhodobacter cell of claim 9 or 10, wherein the monoterpene is selected from the group consisting of beta-pinene, myrcene, alpha-pinene, limonene, linalool, sabinene, bisabolene and geraniol.

12. The Rhodobacter cell of any one of claims 1 to 8, further comprising:
(i) nucleic acid sequences encoding enzymes of a mevalonate pathway for making isoprenyl pyrophosphate (IPP);
(ii) a nucleic acid sequence encoding an enzyme having catalytic activity in the conversion of IPP into farnesyl pyrophosphate (FPP); and
(iii) a nucleic acid sequence encoding an enzyme having sesquiterpene synthase activity in the conversion of FPP into a sesquiterpene, wherein the Rhodobacter cell is free of heterologous enzymes having catalytic activity in the reaction of conversion of two molecules of acetyl-CoA to acetoacyl-CoA.

13. The Rhodobacter cell of claim 12, wherein the enzyme having sesquiterpene synthase activity is selected from the group of enzymes having valencene synthase activity, bisabolene synthase activity, bisabolol synthase activity, bergamotene synthase activity, farnesene synthase activity, zizaene synthase activity, santalene synthase activity, zingiberene synthase activity and patchoulol synthase activity.

14. The Rhodobacter cell of claim 12 or 13, wherein the sesquiterpene is valencene, bisabolene, bisabolol, bergamotene, farnesene, zizaene, santalene zingiberene, or patchoulol.

15. The Rhodobacter cell of claim 12, wherein the Rhodobacter cell exhibits an increased production of a sesquiterpene, preferably zingiberene, using sucrose as carbon source, preferably as sole carbon source, compared to the production of said sesquiterpene, preferably zingiberene, using glucose as carbon source, preferably as sole carbon source.

16. The Rhodobacter cell of claim 9 or 12, further comprising:
(i) a nucleic acid sequence encoding an enzyme having geranylgeranyl pyrophosphate synthase activity;
(ii) a nucleic acid sequence encoding an enzyme having labda-13-en-8-ol diphosphate synthase activity; and
(iii) a nucleic acid sequence encoding an enzyme having sclareol synthase activity,
wherein the Rhodobacter cell preferably exhibits an increased production of sclareol.

17. Method for preparing a monoterpene, a sesquiterpene, a diterpene, a triterpene, a tetraterpene, a flavour, an aroma compound, an UV scavenger, a natural colorant, a natural crop protectant or an isoprenoid moiety containing compound such as coenzyme Q10, comprising culturing a Rhodobacter cell according to any one of the claims 1-16 in a culture medium comprising sucrose as carbon source, preferably sole carbon source, for the monoterpene, sesquiterpene, diterpene, triterpene, tetraterpene, flavour, aroma compound, UV scavenger, natural colorant, natural crop protectant or the isoprenoid moiety containing compound such as coenzyme Q10.

18. Use of the Rhodobacter cell of any one of claims 1 to 16 for the production of a monoterpene, a sesquiterpene, a diterpene, a triterpene, a tetraterpene, a flavour, an aroma compound, an UV scavenger, a natural colorant, a natural crop protectant or for production of an isoprenoid moiety containing compound such as coenzyme Q10, preferably utilizing sucrose as carbon source.
